# EUROPEAN PATENT APPLICATION

(11) **EP 4 631 934 A1**
(43) Date of publication of application: **15.10.2025**
(21) Application number: 23900559.8
(22) Date of filing: 30.11.2023
(51) Int. Cl.: C07D 261/12, C07D 261/14, C07D 413/04

(54) **FLUORINE-CONTAINING ISOXAZOLE COMPOUND AND METHOD FOR PRODUCING SAME**

(30) Priority: 09.12.2022 JP 2022197216
(71) Applicant: Unimatec Co., Ltd., Tokyo 105-0012 (JP)
(72) Inventor: SEINO, Junya, Kitaibaraki-shi, Ibaraki 319-1593 (JP); AOTSU, Rie, Kitaibaraki-shi, Ibaraki 319-1593 (JP); KOKIN, Keisuke, Kitaibaraki-shi, Ibaraki 319-1593 (JP)
(74) Representative: TBK
(86) International application number: PCT/JP2023/042990
(87) International publication number: WO 2024/122444

(57) **Abstract**

The present invention relates to a fluorine-containing isoxazole compound represented by General Formula (A) below. 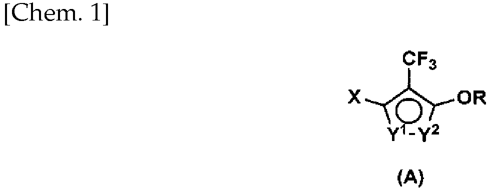 (In the above General Formula (A),
R represents a hydrogen atom or a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

## Description

### Technical Field

The present invention relates to a fluorine-containing isoxazole compound and a method for producing the same.

### Background Art

Compounds having an isoxazole ring or an isoxazolone ring which is a tautomer thereof are known to have various pharmacological effects. Specifically, examples of natural products include ibotenic acid and muscimol. In addition, examples of pharmaceuticals include oxacillin and floxacillin as β-lactam-based antimicrobials, sulfafurazole and sulfamethoxazole as sulfonamide-based antimicrobials, posizolid as oxazolidinone-based antimicrobials, leflunomide and valdecoxib as antirheumatics, tropifexol as a therapeutic agent for liver disease, tivozanib as an anticancer agent, pleconaril as an antiviral agent, isocarboxazid as an antidepressant, glisoxepide as an antidiabetic agent, and danazol as a therapeutic agent for endometriosis. Furthermore, examples of the pesticide include isoxathion as an insecticide and isoxaben as a herbicide.

Based on such a broad pharmacological effect of isoxazoles, in recent years, there has been an interest in introduction of a heteroatom substituent (substituent having a heteroatom) to the 3-position and/or the 5-position. Specifically, Non Patent Literature 1 reports that a compound having an isoxazole ring substituted with a methoxy group at the 3-position and a 1-tetrazolyl group at the 5-position has a cholinesterase inhibitory activity, and is thus studied as a therapeutic agent for Alzheimer's disease.

In addition, there is also an interest in the pharmacological effect of a compound having a trifluoromethyl group at the 4-position of the isoxazole ring. Specifically, Non Patent Literature 2 reports that such a compound has agonist activity for sphingosine-1-phosphate receptor S1P1, and therefore is being studied as a therapeutic agent for autoimmune disease.

From such a viewpoint, it is desired to develop a fluorine-containing isoxazole compound having not only a heteroatom substituent at the 3-position and/or the 5-position of the isoxazole ring but also a trifluoromethyl group at the 4-position of the isoxazole ring in expectation of its usefulness as a therapeutic agent for each disease.

### Document List

### Non Patent Literatures

Non Patent Literature 1: New Journal Of Chemistry, 2015, vol. 39, p. 2028 to 2041
Non Patent Literature 2: Bioorganic & Medicinal Chemistry Letters, 2016, vol. 26, p. 2470 to 2474

### Summary of Invention

### Technical Problem

Therefore, the present inventors have found that a structure having a trifluoromethyl group at the 4-position on an isoxazole ring and having a heteroatom substituent at the 3-position and/or the 5-position can be constructed by reacting a specific raw material, and have completed the present invention.

The present invention provides a novel fluorine-containing isoxazole compound having a trifluoromethyl group at the 4-position on an isoxazolone ring and having a heteroatom substituent at the 3-position and/or the 5-position, and a production method capable of simply producing the fluorine-containing isoxazole compound.

### Solution to Problem

A fluorine-containing isoxazole compound according to the present embodiment is represented by the following General Formula (A). 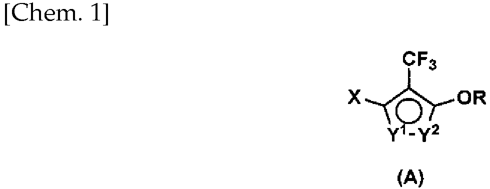 (In the above General Formula (A),
R represents a hydrogen atom or a substituted or unsubstituted hydrocarbon having 1 to 12 carbon atoms,
X represents a halogen atom, -OA¹, -O-NA¹A², -NA¹A², -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

A method for producing a fluorine-containing isoxazole compound according to the present embodiment includes a step of synthesizing a fluorine-containing isoxazole compound represented by the following General Formula (A)
(I) by reacting a fluorine-containing compound selected from a fluorine-containing carbonyl compound, a fluoroisobutene derivative, and a fluoroisobutane derivative, hydroxylamine or a salt thereof, and optionally a compound represented by the following General Formula (5) or a salt thereof, or
(II) by reacting a fluorine-containing compound selected from a fluorine-containing carbonyl compound, a fluoroisobutene derivative, and a fluoroisobutane derivative, an amine salt or an amine compound, and optionally a compound represented by the following General Formula (5) or a salt thereof. 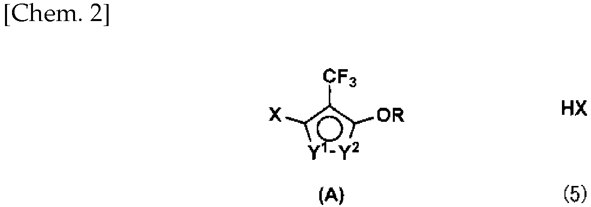 (In the above General Formulae (A) and (5),
   R represents a hydrogen atom or a substituted or unsubstituted hydrocarbon group having 1 to 12 carbon atoms,
   X represents a halogen atom, -OA¹, -O-NA¹A², -NA¹A², -NA¹(OA²), -NA³-NA¹A², -NA⁴, or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   Y¹ and Y² each independently represent N or O, and are different from each other.)

### Effects of Invention

There are provided a novel fluorine-containing isoxazole compound having a trifluoromethyl group at the 4-position on an isoxazolone ring and having a heteroatom substituent at the 3-position and/or the 5-position, and a production method capable of simply producing the fluorine-containing isoxazole compound.

### Description of Embodiments

Hereinafter, embodiments of the present invention will be described in detail. However, the scope of the present invention is not limited to the specific examples described below. The heteroatom substituent means a substituent having a heteroatom, and means a functional group containing at least one heteroatom selected from the group consisting of a nitrogen atom (N), a sulfur atom (S), and an oxygen atom (O).

### (Fluorine-Containing Isoxazole Compound)

The fluorine-containing isoxazole compound in the present embodiment is represented by the following General Formula (A), and has a trifluoromethyl group at the 4-position on the isoxazole ring and a heteroatom substituent at the 3-position and/or the 5-position. 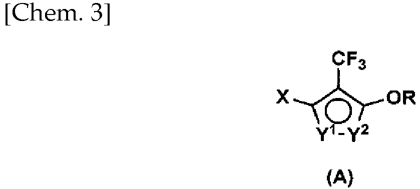 (In the above General Formula (A),
R represents a hydrogen atom or a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

When Y¹ represents N, Y² represents O, and R represents a hydrogen atom, the fluorine-containing isoxazole compound represented by the General Formula (A) is represented by the following General Formula (1). 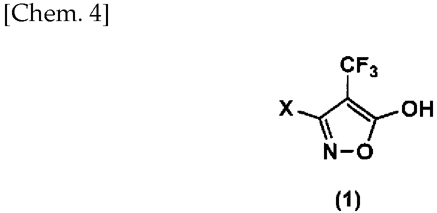 (In the above General Formula (1),
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

When Y¹ represents N, Y² represents O, and R represents a hydrocarbon group having 1 to 12 carbon atoms, the fluorine-containing isoxazole compound represented by the General Formula (A) is represented by the following General Formula (2). 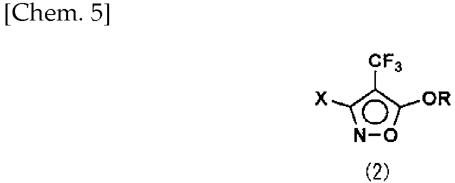 (In the above General Formula (2),
R represents a substituted or unsubstituted hydrocarbon having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

When Y¹ represents O, Y² represents N, and R represents a hydrogen atom, the fluorine-containing isoxazole compound represented by General Formula (A) is represented by the following General Formula (3). 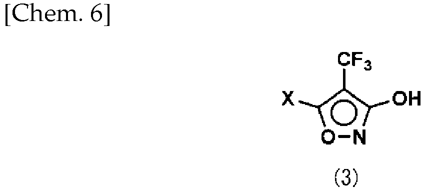 (In the above General Formula (3),
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

When Y¹ represents O, Y² represents N, and R represents a hydrocarbon group having 1 to 12 carbon atoms, the fluorine-containing isoxazole compound represented by the General Formula (A) is represented by the following General Formula (4). 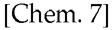 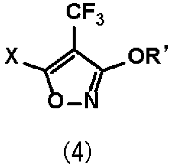 (In the above General Formula (4),
R' represents a substituted or unsubstituted hydrocarbon having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

When R and R' represent a substituted or unsubstituted hydrocarbon group having 1 to 12 carbon atoms, the hydrocarbon group is not particularly limited as long as the hydrocarbon group is a hydrocarbon group consisting of carbon and hydrogen atoms having 1 to 12 carbon atoms, and examples thereof include a chain hydrocarbon group, an aromatic hydrocarbon group, and an alicyclic hydrocarbon group. The chain hydrocarbon group is not particularly limited as long as the total number of carbon atoms is 1 to 12, and may be a linear hydrocarbon group, a branched chain hydrocarbon group, a chain hydrocarbon group having a substituent, or a chain hydrocarbon group having no substituent. When R and R' are aromatic hydrocarbon groups, the aromatic hydrocarbon group is not particularly limited as long as the total number of carbon atoms is 6 to 12, and may be an aromatic hydrocarbon group having a substituent or an aromatic hydrocarbon group having no substituent. Further, the aromatic hydrocarbon group may have a fused polycyclic structure. When R and R' are alicyclic hydrocarbon groups, the alicyclic hydrocarbon group is not particularly limited as long as the total number of carbon atoms is 3 to 12, and may be an alicyclic hydrocarbon group having a substituent or an alicyclic hydrocarbon group having no substituent. Further, the alicyclic hydrocarbon group may have a bridged ring structure.

Examples of the chain hydrocarbon group include alkyl groups such as a methyl group, an ethyl group, a n-propyl group, an i-propyl group, a n-butyl group, an i-butyl group, a sec-butyl group, a ter-butyl group, a pentyl group, a hexyl group, a heptyl group, an octyl group, a nonyl group, a decyl group, an undecyl group, and a dodecyl group;
alkenyl groups such as an ethenyl group, a propenyl group, a butenyl group, a pentenyl group, a hexenyl group, a heptenyl group, an octenyl group, a nonenyl group, a decenyl group, an undecenyl group, and a dodecenyl group; and
alkynyl groups such as an ethynyl group, a propynyl group, a butynyl group, a pentynyl group, a hexynyl group, a heptynyl group, an octynyl group, a nonynyl group, a decynyl group, an undecynyl group, and a dodecynyl group.

Examples of the aromatic hydrocarbon group include a phenyl group and a naphthyl group.

Examples of the alicyclic hydrocarbon group include a saturated or unsaturated cyclic hydrocarbon group, and examples of the cyclic hydrocarbon group include a cyclopropyl group, a cyclobutyl group, a cyclohexyl group, a cyclopentyl group, an adamantyl group, and a norbornyl group.

When the chain hydrocarbon group has a substituent, one of hydrogen atoms of the chain hydrocarbon group may be substituted with an alkoxyl group or an aralkyl group. The alkoxyl group is preferably substituted with an alkoxyl group having 1 to 6 carbon atoms, and examples thereof include a methoxy group, an ethoxy group, an n-propoxy group, an isopropoxy group, an n-butoxy group, an isobutoxy group, a sec-butoxy group, a tet-butoxy group, an n-pentoxy group, and an n-hexyloxy group. As the aralkyl group, an alkyl group having 1 to 6 carbon atoms is preferably substituted with an aryl group, and examples thereof include a benzyl group, a phenylethyl group, a phenylpropyl group, and a naphthylmethyl group.

When the aromatic hydrocarbon group and the alicyclic hydrocarbon group have a substituent, examples of the substituent include the above-described alkyl group having 1 to 6 carbon atoms and alkoxyl group.

In X, the halogen atom is F, Cl, Br, or I, preferably F or Cl.

In X, A¹, A², and A³ included in -OA¹, -O-NA¹A², -NA¹A², -NA¹(OA²), and -NA³-NA¹A² each independently represent a substituted or unsubstituted hydrocarbon group having 1 to 12 carbon atoms or a heterocycle. When X represents -O-NA¹A², -NA¹A², -NA¹(OA²), and -NA³-NA¹A², A¹, A², and A³ may be the same or different from each other. When A¹, A², and A³ represent a substituted or unsubstituted hydrocarbon group having 1 to 12 carbon atoms, A¹, A², and A³ are the same as the hydrocarbon group having 1 to 12 carbon atoms defined by R and R' above, and can be, for example, a hydrocarbon group having 1 to 12 carbon atoms among R and R' above. The hydrocarbon group having 1 to 12 carbon atoms is preferably a chain hydrocarbon group having 1 to 12 carbon atoms, more preferably a chain hydrocarbon group having 1 to 10 carbon atoms, further preferably an alkyl group having 1 to 10 carbon atoms, and particularly preferably an alkyl group having 1 to 4 carbon atoms.

In X, the heterocycle may be optionally substituted, and is a monocyclic, bicyclic, or polycyclic heterocycle, which is a heterocyclic group containing, as ring atoms, at least one heteroatom selected from the group consisting of nitrogen atoms (N), sulfur atoms (S), and oxygen atoms (O). The heterocyclic group may have a plurality of identical or different heteroatoms. The skeleton of each heterocycle preferably contains at least one nitrogen atom as a heteroatom. One of the heteroatoms contained in the heterocyclic group, preferably N, is directly bonded to the isoxazole ring of the fluorine-containing isoxazole compound, and is substituted with the isoxazole ring as a heteroatom substituent. The heterocycle may be a fused ring, or may be any of an alicyclic heterocycle and an aromatic heterocycle, or may be a combination thereof.

The monocyclic heterocycle is preferably a 3 to 12 membered ring, and more preferably a 5 to 9 membered ring. When the heterocycle is a monocyclic heterocycle, up to 5 heteroatoms may be included. Preferably, each heteroatom is individually selected from O, S, and N, and at least one of the heteroatoms is N. Examples of the monocyclic heterocycle include pyrrolidine, pyrroline, pyrrole, pyrazolidine, imidazolidine, pyrazoline, imidazoline, imidazole, pyrazole, triazole, tetrazole, (iso)oxazole, (iso)oxadiazole, (iso)thiazole, thiadiazole, pyridine, pyrrolidine, piperidine, piperazine, pyridazine, pyrimidine, pyrazine, triazine, thienopyridine, piperazinone, morpholine, thiomorpholine, thiomorpholine dioxide, oxazine, thiazine, azocane, azocine, azonan, azonin, and derivatives thereof.

The bicyclic heterocycle is preferably a 7 to 14 membered ring, and more preferably a 8 to 10 membered ring. A spiro ring may be contained in the bicyclic heterocycle. When the heterocycle is a bicyclic heterocycle, up to 10 heteroatoms may be included. Preferably, each heteroatom is individually selected from O, S, and N, and at least one of the heteroatoms is N. Examples of the bicyclic heterocycle include (iso)indole, aza (iso)indole, (aza)indazole, (aza)benzimidazole, (aza)benztriazole, hydrothienopyridine, (iso)quinoline, hydro(iso)quinoline, hydrofuropyridine, and derivatives thereof.

The polycyclic heterocycle is preferably a 9 to 30 membered ring, and more preferably a 12 to 26 membered ring. Further, spiro ring may be contained in the polycyclic heterocycle. When the heterocycle is a polycyclic heterocycle, up to 15 heteroatoms may be included. Preferably, each heteroatom is individually selected from O, S, and N, and at least one of the heteroatoms is N. Examples of the polycyclic heterocycle include carbazole, phenazine, phenoxazine, phenothiazine, benzoindole, pyrroloquinoline, acridine, and derivatives thereof.

When the heterocycle has a substituent, examples of the substituent include oxygen-containing substituents such as a halogen atom, a C₁-C₁₀ hydrocarbon group, a hydroxy group, an alkoxy group, a carbonyl group, and a carboxy group; nitrogen-containing substituents such as an amino group, a cyano group, and a nitro group; and sulfur-containing substituents such as a sulfanyl group, a sulfoxy group, and a sulfone group.

When A¹, A², and A³ are heterocycles, A¹, A², and A³ are the same as the heterocycle defined as X above. When X is -O-NA¹A² or -NA¹A², A¹ may be a substituted or unsubstituted hydrocarbon group having 1 to 12 carbon atoms, and A² may be a heterocycle. When X is -NA¹(OA²), A¹ may be a substituted or unsubstituted hydrocarbon group having 1 to 12 carbon atoms, A² may be a heterocycle, A¹ may be a heterocycle, and A² may be a substituted or unsubstituted hydrocarbon group having 1 to 12 carbon atoms. When X represents -NA³-NA¹A², A¹ and A³ may be a substituted or unsubstituted hydrocarbon group having 1 to 12 carbon atoms, A² may be a heterocycle, A¹ may be a substituted or unsubstituted hydrocarbon group having 1 to 12 carbon atoms, and A² and A³ may be a heterocycle.

In X, A⁴ included in -NA⁴ represents an alkylene group represented by =CₙH₂ₙ, and n is an integer of 1 to 20. The alkylene group represented by =CₙH₂ₙ may be linear or may have a branched structure. n is preferably 3 to 15 and more preferably 5 to 10.

Since the fluorine-containing isoxazole compound in the present embodiment has a specific substituent (-X, -CF₃, -OR, or -OR') on the isoxazole ring, from the viewpoint of structural expandability, excellent effects can be achieved, and in particular, further improvement in pharmacological activity can be expected. In addition, since the 3-position, the 4-position, and the 5-position on the isoxazole ring can have different substituents, derivatization to an asymmetric structure can be easily performed, and use as an intermediate can also be expected. More specifically, a derivative can be obtained by modifying - OR or -OR' by reacting a fluorine-containing isoxazole compound under acidic conditions. When the substituent X on the isoxazole ring is a halogen atom, a derivative can be obtained by modifying a halogen atom by reacting a fluorine-containing isoxazole compound under basic conditions. Furthermore, the fluorine-containing isoxazole compound in the present embodiment is also useful, for example, in the field of electronic materials such as organic semiconductors and liquid crystals.

### (Method for Producing Fluorine-Containing Isoxazole Compound)

The method for producing a fluorine-containing isoxazole compound in the present embodiment includes: a step of synthesizing a fluorine-containing isoxazole compound represented by General Formula (A) by (I) reacting a fluorine-containing compound selected from the group consisting of fluorine-containing carbonyl compounds, fluoroisobutene derivatives, and fluoroisobutane derivatives, hydroxylamine or a salt thereof, and optionally a compound represented by the following General Formula (5) or a salt thereof, or by (II) reacting a fluorine-containing compound selected from fluorine-containing carbonyl compounds, fluoroisobutene derivatives, and fluoroisobutane derivatives, an amine salt or an amine compound, and optionally a compound represented by the following General Formula (5) or a salt thereof. 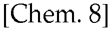 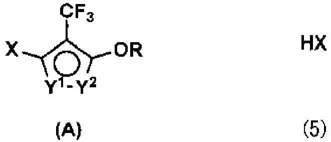 (In the above General Formulae (A) and (5),
R represents a hydrogen atom or a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

Such a fluorine-containing isoxazole compound represented by General Formula (A) is synthesized, for example, by the following method.

A first embodiment for producing the fluorine-containing isoxazole compound in the present embodiment includes a step of (a) obtaining a fluorine-containing isoxazole compound represented by the following General Formula (1) by reacting a fluorine-containing carbonyl compound represented by the following General Formula (6) with hydroxylamine represented by the following General Formula (7) or a salt thereof. 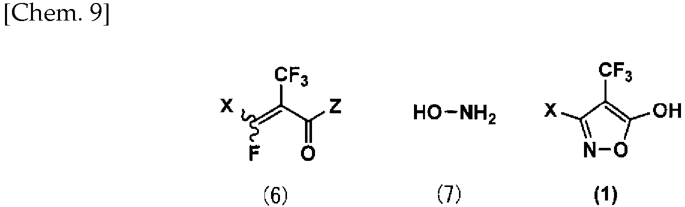 (In the above General Formulae (1) and (6),
X represents a halogen atom, -OA¹, -O-NA¹A², -NA¹A², -NA¹(OA²), -NA³-NA¹A², -NA⁴, or a heterocycle,
Z represents -OA⁵, -O-NA⁵A⁶, -NA⁵A⁶, -NA⁵(OA⁶), or -NA⁷-NA⁵A⁶,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20,
A⁵, A⁶, and A⁷ each independently represent a substituted or unsubstituted hydrocarbon group having 1 to 12 carbon atoms, and
a wavy bond indicates a single bond representing stereoisomerism of (E) or (Z).)

A second embodiment for producing the fluorine-containing isoxazole compound in the present embodiment includes a step of (b) obtaining a fluorine-containing isoxazole compound represented by the following General Formula (1) by reacting a fluorine-containing carbonyl compound represented by the following General Formula (8) with a compound represented by the following General Formula (5) or a salt thereof, and hydroxylamine represented by the following General Formula (7) or a salt thereof. 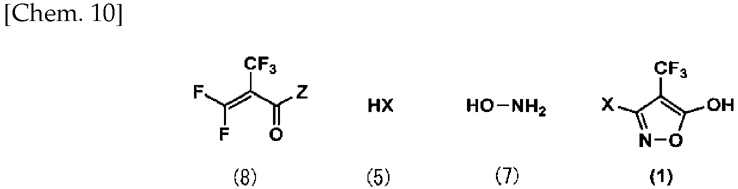 (In the above General Formulae (1), (5), and (8),
X represents a halogen atom, -OA¹, -O-NA¹A², -NA¹A², -NA¹(OA²), -NA³-NA¹A², -NA⁴, or a heterocycle,
Z represents -OA⁵, -O-NA⁶A⁷, -NA⁵A⁶, -NA⁵(OA⁶), or -NA⁷-NA⁵A⁶, and A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
A⁵, A⁶, and A⁷ each independently represent a substituted or unsubstituted hydrocarbon group having 1 to 12 carbon atoms.)

A third embodiment for producing the fluorine-containing isoxazole compound in the present embodiment includes a step of (c) obtaining a fluorine-containing isoxazole compound represented by the following General Formula (1) by reacting a fluorine-containing carbonyl compound represented by the following General Formula (9) with a compound represented by the following General Formula (5) or a salt thereof in the presence of a base, and then further reacting the obtained reaction product with hydroxylamine represented by the following General Formula (7) or a salt thereof. 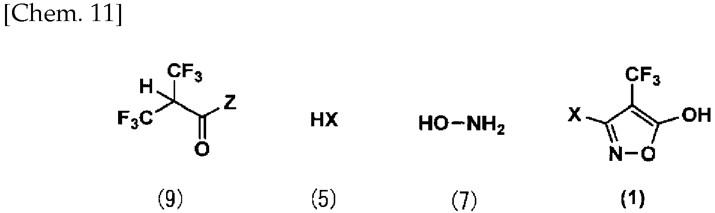 (In the above General Formulae (1), (5), and (9),
X represents a halogen atom, -OA¹, -O-NA¹A², -NA¹A², -NA¹(OA²), -NA³-NA¹A², -NA⁴, or a heterocycle,
Z represents -OA⁵, -O-NA⁶A⁷, -NA⁵A⁶, -NA⁵(OA⁶), or -NA⁷-NA⁵A⁶,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
A⁵, A⁶, and A⁷ each independently represent a substituted or unsubstituted hydrocarbon group having 1 to 12 carbon atoms.)

A fourth embodiment for producing the fluorine-containing isoxazole compound in the present embodiment includes a step of (d) obtaining a fluorine-containing isoxazole compound represented by the following General Formula (1) by reacting a fluorine-containing carbonyl compound represented by the following General Formula (10) with an alcohol, then reacting the obtained reaction product with a compound represented by the following General Formula (5) or a salt thereof in the presence of a base, and then reacting the obtained reaction product with hydroxylamine represented by the following General Formula (7) or a salt thereof. 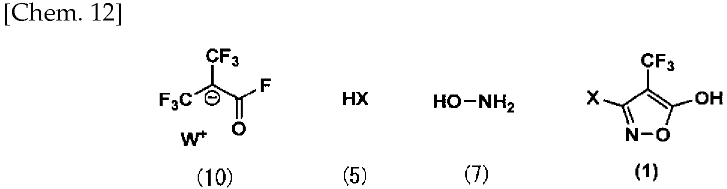 (In the above General Formulae (1), (5), and (10),
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

A fifth embodiment for producing the fluorine-containing isoxazole compound in the present embodiment includes a step of (e) obtaining a fluorine-containing isoxazole compound represented by the following General Formula (1) by carbonylating a fluoroisobutene derivative represented by the following General Formula (11) in the presence of a nucleophile, further reacting the fluoroisobutene derivative with an alcohol, then reacting the obtained reaction product with a compound represented by the following General Formula (5) or a salt thereof in the presence of a base, and then reacting the reaction product with hydroxylamine represented by the following General Formula (7) or a salt thereof. 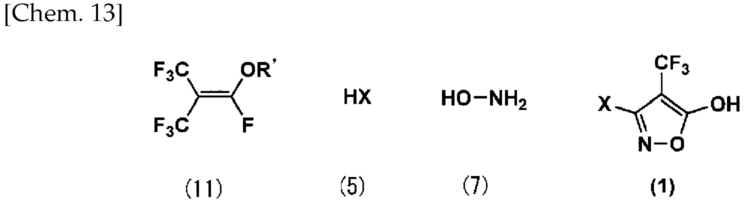 (In the above General Formulae (1), (5), and (11),
R' represents a substituted or unsubstituted hydrocarbon group having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

A sixth embodiment for producing the fluorine-containing isoxazole compound in the present embodiment includes a step of (f) obtaining a fluorine-containing isoxazole compound represented by the following General Formula (1) by carbonylating a compound obtained by an elimination reaction of the fluoroisobutane derivative represented by the following General Formula (12) in the presence of a nucleophile, further reacting the fluoroisobutane derivative with an alcohol, then reacting the obtained reaction product with a compound represented by the following General Formula (5) or a salt thereof in the presence of a base, and then reacting the reaction product with hydroxylamine represented by the following General Formula (7) or a salt thereof. 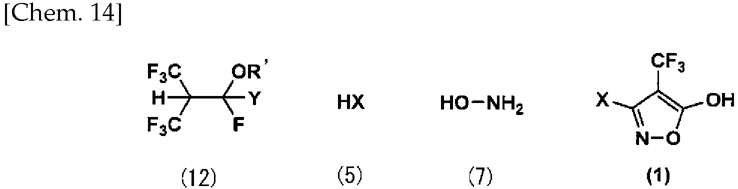 (In the above General Formulae (1), (5), and (12), R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

A seventh embodiment for producing the fluorine-containing isoxazole compound in the present embodiment includes a step of (g) obtaining a fluorine-containing isoxazole compound represented by the following General Formula (2) by reacting a fluorine-containing carbonyl compound represented by the following General Formula (13) with an amine salt or an amine compound. 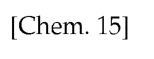 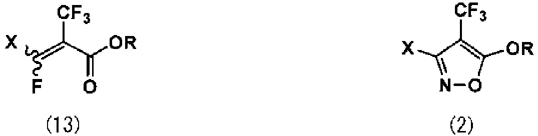 (In the above General Formulae (2) and (13),
R represents a substituted or unsubstituted hydrocarbon group having 1 to 12 carbon atoms,
X represents -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
a wavy bond indicates a single bond representing stereoisomerism of (E) or (Z).)

An eighth embodiment for producing the fluorine-containing isoxazole compound in the present embodiment includes a step of (h) obtaining a fluorine-containing isoxazole compound represented by the following General Formula (2) by reacting a fluorine-containing carbonyl compound represented by the following General Formula (14) with an amine salt or an amine compound, and optionally a compound represented by the following General Formula (5) or a salt thereof. 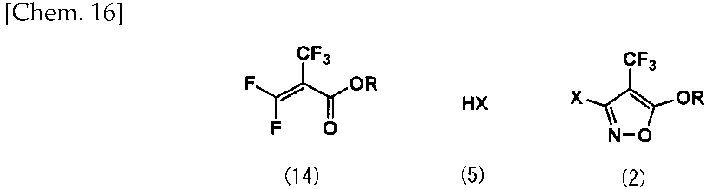 (In the above General Formulae (2) and (14),
R represents a substituted or unsubstituted hydrocarbon having 1 to 12 carbon atoms,
X represents a halogen atom, -OA¹, O-NA¹A², -NA¹(OA²), -NA¹A², -NA³-NA¹A², -NA⁴ or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

A ninth embodiment for producing the fluorine-containing isoxazole compound in the present embodiment includes a step of (i) obtaining a fluorine-containing isoxazole compound represented by the following General Formula (2) by reacting a fluorine-containing carbonyl compound represented by the following General Formula (15) with a compound represented by the following General Formula (5) or a salt thereof in the presence of a base, and then reacting the obtained reaction product with an amine salt or an amine compound. 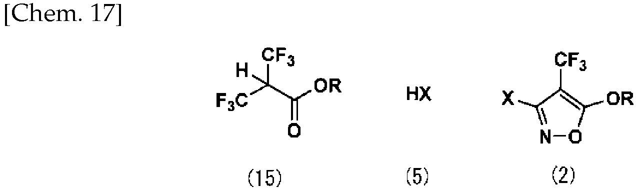 (In the above General Formulae (2), (5), and (15),
R represents a substituted or unsubstituted hydrocarbon group having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

A tenth embodiment for producing the fluorine-containing isoxazole compound in the present embodiment includes a step of (j) obtaining a fluorine-containing isoxazole compound represented by the following General Formula (2) by reacting a fluorine-containing carbonyl compound represented by the following General Formula (10) with an alcohol, then reacting the obtained reaction product with a compound represented by the following General Formula (5) or a salt thereof in the presence of a base, and then reacting the reaction product with an amine salt or an amine compound. 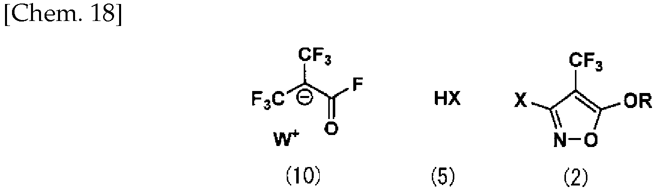 (In the above General Formulae (2), (5), and (10),
R represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

An eleventh embodiment for producing the fluorine-containing isoxazole compound in the present embodiment includes a step of (k) obtaining a fluorine-containing isoxazole compound represented by the following General Formula (2) by carbonylating a fluoroisobutene derivative represented by the following General Formula (11) in the presence of a nucleophile, further reacting the fluoroisobutene derivative with an alcohol, then reacting the obtained reaction product with a compound represented by the following General Formula (5) or a salt thereof in the presence of a base, and then reacting an amine salt or an amine compound. 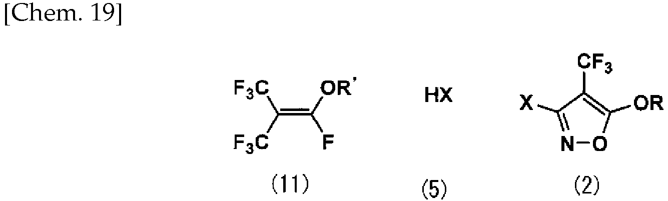 (In the above General Formulae (2), (5), and (11),
R and R' each independently represent a substituted or unsubstituted hydrocarbon having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

A twelfth embodiment for producing the fluorine-containing isoxazole compound in the present embodiment includes a step of (l) obtaining a fluorine-containing isoxazole compound represented by the following General Formula (2) by carbonylating a compound obtained by an elimination reaction of the fluoroisobutane derivative following General Formula (12) in the presence of a nucleophile, further reacting the fluoroisobutane derivative with an alcohol, then reacting the obtained reaction product with a compound represented by the following General Formula (5) or a salt thereof in the presence of a base, and then reacting an amine salt or an amine compound. 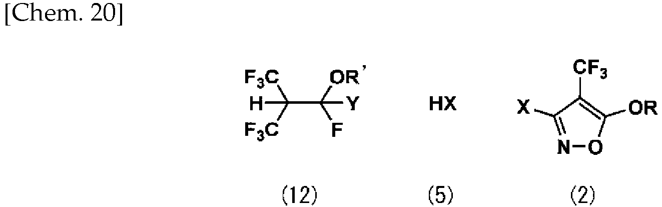 (In the above General Formulae (2), (5), and (12),
R and R' each independently represent a substituted or unsubstituted hydrocarbon group having 1 to 12 carbon atoms,
X represents a halogen atom, -OA¹, -O-NA¹A², -NA¹A², -NA¹(OA²), -NA³-NA¹A²-NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

A thirteenth embodiment for producing the fluorine-containing isoxazole compound in the present embodiment includes a step of (m) obtaining a fluorine-containing isoxazole compound represented by the following General Formula (3) by reacting a fluorine-containing carbonyl compound represented by the following General Formula (10) with hydroxylamine represented by the following General Formula (7) or a salt thereof, and further reacting the obtained reaction product with a compound represented by the following General Formula (5) or a salt thereof. 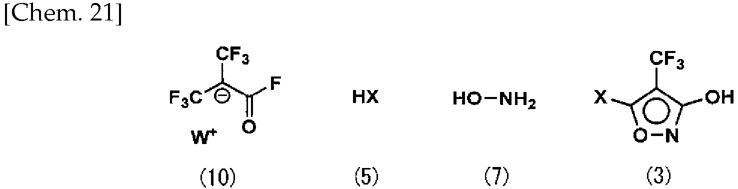 (In the above General Formulae (3), (5), and (10),
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

A fourteenth embodiment for producing the fluorine-containing isoxazole compound in the present embodiment includes a step of (n) obtaining a fluorine-containing isoxazole compound represented by the following General Formula (3) by carbonylating a fluoroisobutene derivative represented by the following General Formula (11) in the presence of a nucleophile, further reacting the fluoroisobutene derivative with hydroxylamine represented by the following General Formula (7) or a salt thereof, and then reacting the obtained reaction product with a compound represented by the following General Formula (5) or a salt thereof. 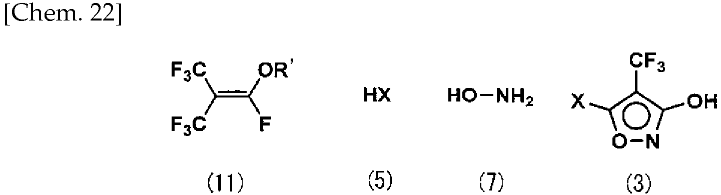 (In the above General Formulae (3), (5), and (11),
R' represents a substituted or unsubstituted hydrocarbon having 1 to 12 carbon atoms,
X represents a halogen atom, -OA¹, O-NA¹A², -NA¹A², -NA¹(OA²), -NA³-NA¹A², -NA⁴ or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

A fifteenth embodiment for producing the fluorine-containing isoxazole compound in the present embodiment includes a step of (o) obtaining a fluorine-containing isoxazole compound represented by the following General Formula (3) by carbonylating a fluoroisobutane derivative represented by the following General Formula (12) in the presence of a nucleophile, further reacting the fluoroisobutane derivative with hydroxylamine represented by the following General Formula (7) or a salt thereof, and then reacting the obtained reaction product with a compound represented by the following General Formula (5) or a salt thereof. 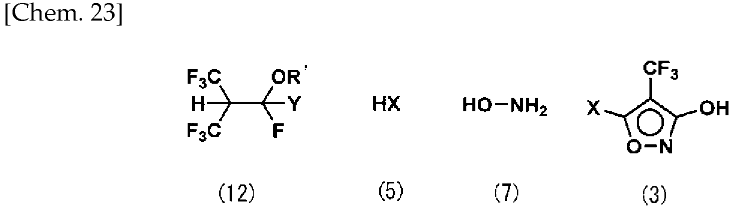 (In the above General Formulae (3), (5), and (12),
R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A²-NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

A sixteenth embodiment for producing the fluorine-containing isoxazole compound in the present embodiment includes a step of (p) obtaining a fluorine-containing isoxazole compound represented by the following General Formula (4) by reacting a fluoroisobutene derivative represented by the following General Formula (11) with a compound represented by the following General Formula (5) or a salt thereof, and hydroxylamine represented by the following General Formula (7) or a salt thereof. [Chem. 24] 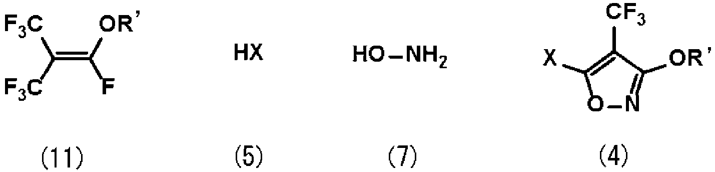 (In the above General Formulae (4), (5), and (11),
R' represents a substituted or unsubstituted hydrocarbon having 1 to 12 carbon atoms,
X represents a halogen atom, -OA¹, O-NA¹A², -NA¹(OA²), -NA¹A², -NA³-NA¹A², -NA⁴ or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

A seventeenth embodiment for producing the fluorine-containing isoxazole compound in the present embodiment includes a step of (q) obtaining a fluorine-containing isoxazole compound represented by the following General Formula (4-1) by reacting a fluoroisobutene derivative represented by the following General Formula (11) with hydroxylamine represented by the following General Formula (7) or a salt thereof. 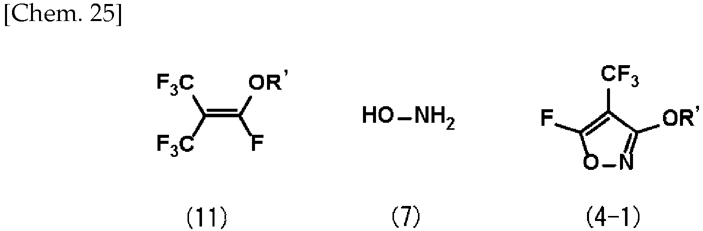 (In the above General Formulae (4-1) and (11),
R' represents a substituted or unsubstituted hydrocarbon group having 1 to 12 carbon atoms.)

An eighteenth embodiment for producing the fluorine-containing isoxazole compound in the present embodiment includes a step of (r) obtaining a fluorine-containing isoxazole compound represented by the following General Formula (4) by reacting a fluoroisobutane derivative represented by the following General Formula (12) with a compound represented by the following General Formula (5) or a salt thereof, and hydroxylamine represented by the following General Formula (7) or a salt thereof. 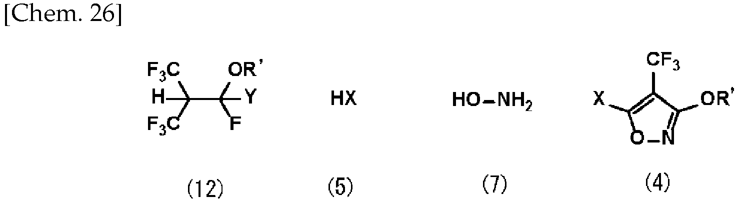 (In the above General Formulae (4), (5), and (12),
R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

A nineteenth embodiment for producing the fluorine-containing isoxazole compound in the present embodiment includes a step of (s) obtaining a fluorine-containing isoxazole compound represented by the following General Formula (4-1) by reacting a fluoroisobutane derivative represented by the following General Formula (12) with hydroxylamine represented by the following General Formula (7) or a salt thereof. 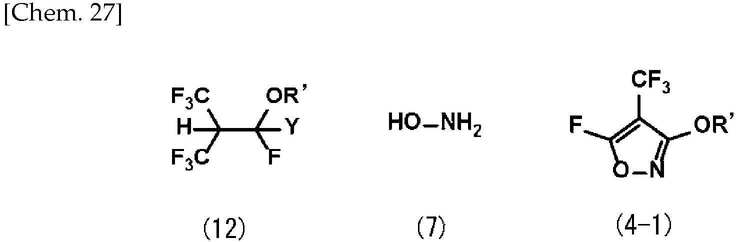 (In the above General Formulae (4-1) and (12),
R' represents a substituted or unsubstituted hydrocarbon group having 1 to 12 carbon atoms,
Y represents a halogen atom, -OB¹, -SOₘB¹, or -NB¹B²,
m is an integer of 0 to 3, and
B¹ and B² each independently represent a substituted or unsubstituted hydrocarbon group having 1 to 12 carbon atoms.)

In the fluorine-containing compound, the fluorine-containing carbonyl compound is preferably a compound represented by the above General Formula (6), (8), (9), (10), (13), (14) or (15), the fluoroisobutene derivative is preferably a compound represented by the above General Formula (11), and the fluoroisobutane derivative is preferably a compound represented by the above General Formula (12).

In the above General Formulae (5), (6), and (13), A¹, A², and A³ and the heterocycle are the same as those defined in the fluorine-containing isoxazole compounds represented by the General Formulae (A) and (1) to (4) described above. In addition, in the above General Formulae (4-1), (11), and (12), R' is the same as those defined in the fluorine-containing isoxazole compound represented by the General Formula (4) described above, and the substituted or unsubstituted hydrocarbon group having 1 to 12 carbon atoms in R' is preferably an alkyl group having 1 to 10 carbon atoms.

In the above General Formulae (6), (8), and (9), A⁵, A⁶, and A⁷ are the same as the substituted or unsubstituted hydrocarbon group having 1 to 12 carbon atoms defined in R and R' described above, and the substituted or unsubstituted hydrocarbon group having 1 to 12 carbon atoms in A⁵, A⁶, and A⁷ is preferably an alkyl group having 1 to 10 carbon atoms.

In the above General Formula (12), B¹ and B² are the same as the substituted or unsubstituted hydrocarbon group having 1 to 12 carbon atoms as defined in R and R' described above.

In General Formula (12), Y represents a halogen atom, -OB¹, -SOₘB¹, or - NB¹B².

In Y, the halogen atom is F, Cl, Br, or I, preferably F or Cl.

In Y, B¹ contained in -OB¹ and -SOₘB¹ represents a substituted or unsubstituted hydrocarbon group having 1 to 12 carbon atoms, and is the same as the hydrocarbon group having 1 to 12 carbon atoms defined by R and R' described above. In addition, m is an integer of 0 to 3, preferably an integer of 0 to 2, and more preferably an integer of 0 to 1.

In Y, B¹ and B² contained in -NB¹B² are each independently a substituted or unsubstituted hydrocarbon group having 1 to 12 carbon atoms. B¹ and B² may be the same or different from each other. B¹ and B² are the same as the hydrocarbon group having 1 to 12 carbon atoms defined by R and R' described above.

The reaction of the above (a) in the first embodiment is represented by the following Reaction Formula (A) as a whole.

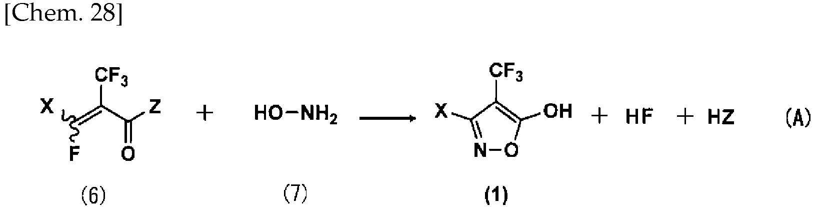

The reaction of the above (b) in the second embodiment is represented by the following Reaction Formula (B) as a whole.

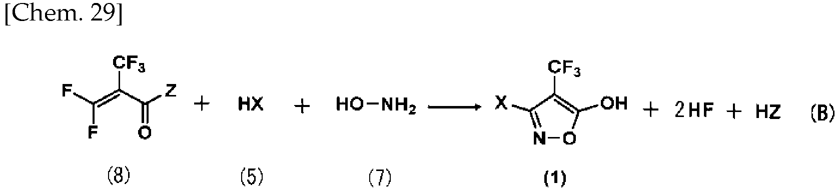

The reaction of the above (c) in the third embodiment is represented by the following Reaction Formula (C) as a whole.

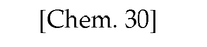

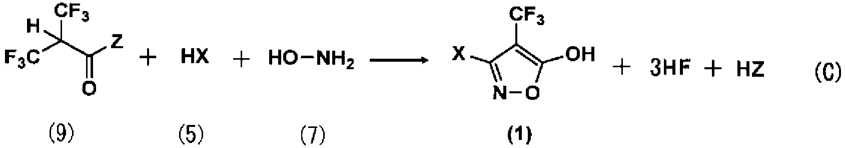

The reaction of the above (d) in the fourth embodiment is represented by the following Reaction Formula (D) as a whole. Furthermore, in the following reaction formula, "WF" represents a salt of a cation W⁺ and F⁻.

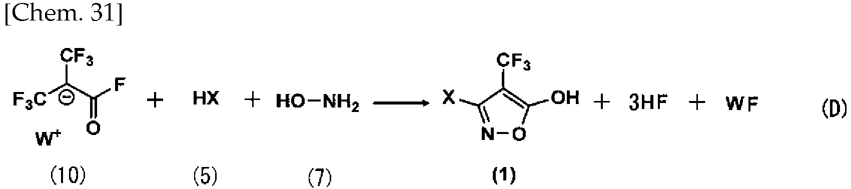

The reaction of the above (e) in the fifth embodiment is represented by the following Reaction Formula (E) as a whole. Furthermore, in the following reaction formula, the nucleophile may be represented as "Nu" for convenience. "NuR'F" represents a salt of a cation of a nucleophile in which R' is substituted and F⁻.

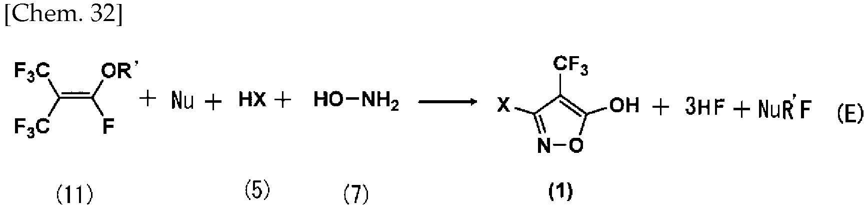

The reaction of the above (f) in the sixth embodiment is represented by the following Reaction Formula (F) as a whole.

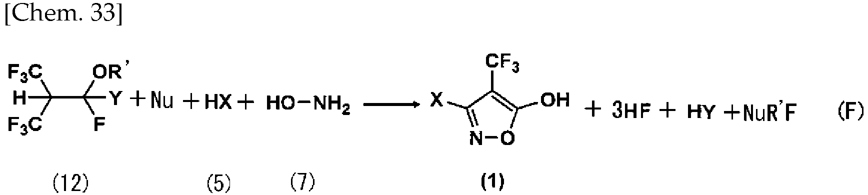

The reaction of the above (g) in the seventh embodiment is represented by the following Reaction Formula (G) as a whole. Furthermore, in the following Reaction Formulae (G) to (O), the amine salt or the amine compound may be represented as "N compound" for convenience.

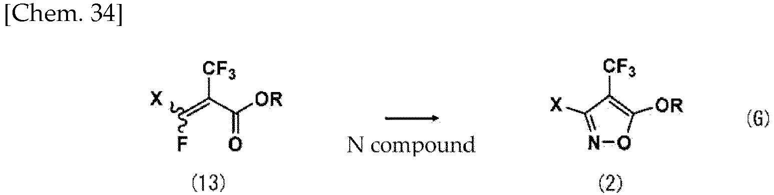

The reaction of the above (h) in the eighth embodiment is represented by the following Reaction Formula (H) as a whole. In the reaction of (h), when the compound represented by General Formula (5) or a salt thereof is not used, X represents a fluorine atom (F) in the fluorine-containing isoxazole compound represented by General Formula (2).

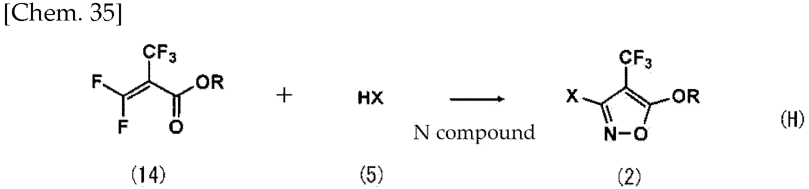

The reaction of the above (i) in the ninth embodiment is represented by the following Reaction Formula (I) as a whole.

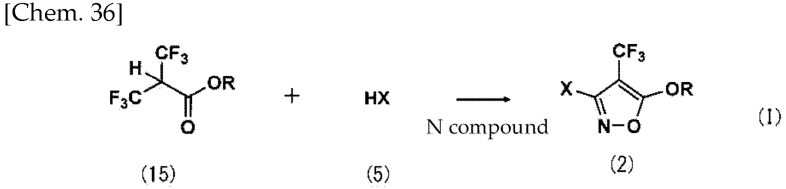

The reaction of the above (j) in the tenth embodiment is represented by the following Reaction Formula (J) as a whole.

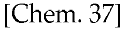

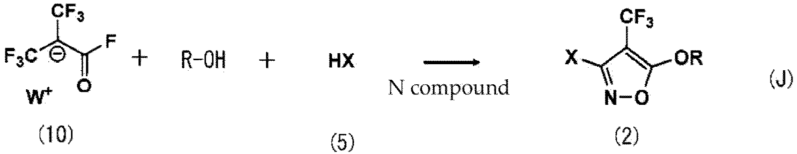

The reaction of the above (k) in the eleventh embodiment is represented by the following Reaction Formula (K) as a whole.

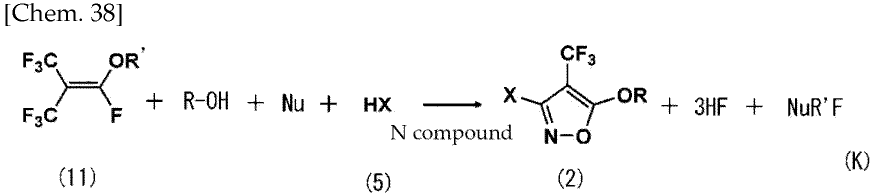

The reaction of the above (l) in the twelfth embodiment is represented by the following Reaction Formula (L) as a whole.

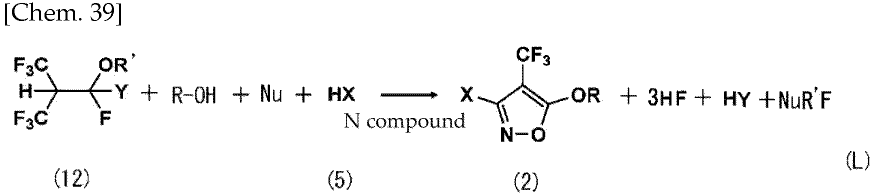

The reaction of the above (m) in the thirteenth embodiment is represented by the following Reaction Formula (M) as a whole.

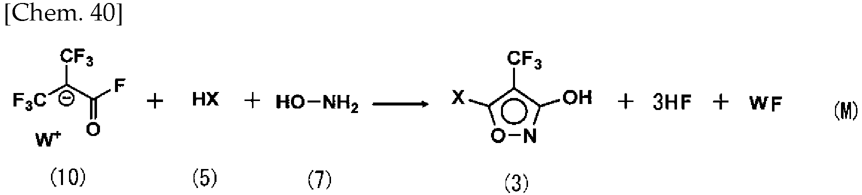

The reaction of the above (n) in the fourteenth embodiment is represented by the following Reaction Formula (N) as a whole.

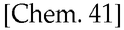

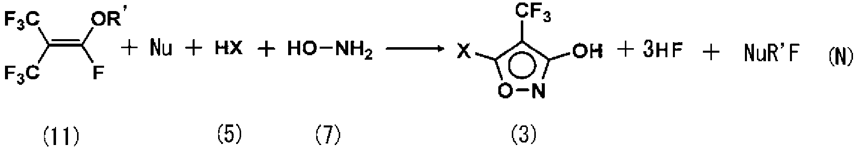

The reaction of the above (o) in the fifteenth embodiment is represented by the following Reaction Formula (O) as a whole.

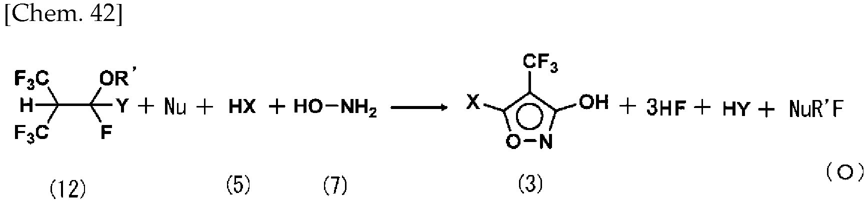

The reaction of the above (p) in the sixteenth embodiment is represented by the following Reaction Formula (P) as a whole.

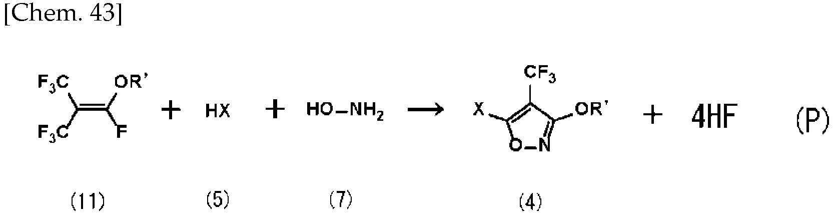

The reaction of the above (q) in the seventeenth embodiment is represented by the following Reaction Formula (Q) as a whole.

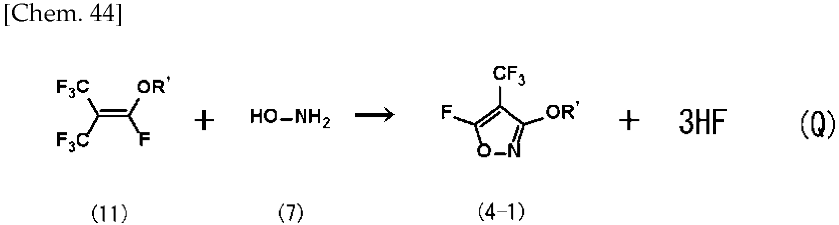

The reaction of the above (r) in the eighteenth embodiment is represented by the following Reaction Formula (R) as a whole.

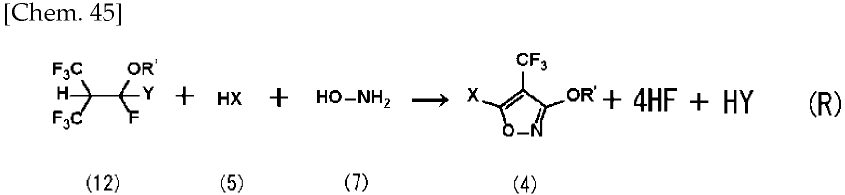

The reaction of the above (s) in the nineteenth embodiment is represented by the following Reaction Formula (S) as a whole.

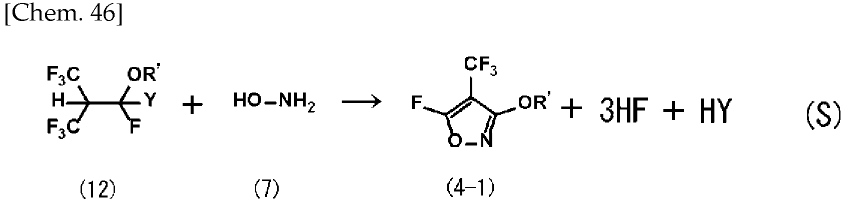

In each of the above reactions, the hydroxylamine represented by General Formula (7) and the compound represented by General Formula (5) may each be in the form of a salt. When the hydroxylamine represented by General Formula (7) is in the form of a salt, an example can be given in which the moiety (-NH₂) part constituting the amino group of the hydroxylamine is cationized to form (-NH₃⁺), and forms a salt with a counter ion. In addition, when the compound represented by General Formula (5) is in the form of a salt, an example can be given in which the H moiety of the compound is cationized to form (H⁺), and a salt is formed with a counter ion. The counter ion is not particularly limited as long as the counter ion is a monovalent anion, and examples thereof include halide ions such as F⁻, Cl⁻, Br, and I⁻, trifluoroacetate anion, p-toluenesulfonate anion, trifluoromethanesulfonate anion, nonafluorobutanesulfonate, bis(trifluoromethylsulfonyl)imide anion, and tetrafluoroborate anion.

When an amine salt or an amine compound is used in each of the above reactions, examples of the amine salt and the amine compound include an amine salt of a nitrogen-containing heterocyclic cation in which one amino group (-NH₂) is bonded to a nitrogen atom within a heterocycle, or a quaternary ammonium cation in which one amino group (-NH₂) is bonded to a nitrogen atom, with an anion selected from halogen, sulfuric acid, phosphoric acid, sulfonic acid, trifluoroacetic acid, tetrafluoroborate, tetraphenylborate, hexafluoroborate, and sulfonylimide acid; a primary amine, hydroxylamine, or amine salt having an anionic substituent or anion selected from halogen, sulfuric acid, phosphoric acid, sulfonic acid, and trifluoroacetic acid; or a quaternary ammonium salt substituted with a hydrocarbon group selected from alkyl groups and aryl groups.

In the nitrogen-containing heterocyclic cation, at least one nitrogen atom may be contained in the heterocycle as a heteroatom, and may be optionally substituted. In addition, the nitrogen atom on the nitrogen-containing heterocycle is directly bonded to one amino group (-NH₂), and the nitrogen-containing heterocyclic cation is preferably a monocyclic heterocycle. On the nitrogen-containing heterocycle, at least one heteroatom selected from the group consisting of a nitrogen atom, a sulfur atom, and an oxygen atom (O) may be further contained as an atom constituting the ring. The nitrogen-containing heterocycle is preferably an aromatic heterocycle, and the number of ring members is preferably a 5 to 8 membered ring, and more preferably a 5 or 6 membered ring. Examples of such a nitrogen-containing heterocycle include pyridine, 4-dimethylaminopyridine, and 1-methylimidazole.

In the quaternary ammonium cation in which one amino group (-NH₂) is bonded to a nitrogen atom, the nitrogen atom of the quaternary ammonium cation is preferably bonded to a hydrocarbon group selected from an alkyl group and an aryl group as the other substituent. The alkyl group preferably has 1 to 12 carbon atoms, and may be linear or branched. Examples of such an alkyl group include a methyl group, an ethyl group, an n-propyl group, an i-propyl group, an n-butyl group, an i-butyl group, a sec-butyl group, a ter-butyl group, a pentyl group, a hexyl group, a heptyl group, an octyl group, a nonyl group, a decyl group, an undecyl group, and a dodecyl group. The aryl group preferably has 6 to 12 carbon atoms, and may be substituted with a methyl group or the like. Examples of such an aryl group include a tolyl group and a mesityl group.

In the primary amine, hydroxylamine, or amine salt having the anionic substituent or anion described above, the anionic substituent or anion is preferably directly bonded to the nitrogen atom constituting the amine. In addition, in the hydroxylamine having an anionic substituent or anion, an anionic substituent or anion is preferably directly bonded to an oxygen atom (-O-NH₂) constituting the hydroxylamine.

In the quaternary ammonium salt, the nitrogen atom of the quaternary ammonium salt is substituted with a hydrocarbon group selected from an alkyl group and an aryl group, and is not bonded to an amino group (-NH₂). The alkyl group preferably has 1 to 12 carbon atoms, and may be linear or branched. Examples of such an alkyl group include a methyl group, an ethyl group, an n-propyl group, an i-propyl group, an n-butyl group, an i-butyl group, a sec-butyl group, a ter-butyl group, a pentyl group, a hexyl group, a heptyl group, an octyl group, a nonyl group, a decyl group, an undecyl group, and a dodecyl group. The aryl group preferably has 6 to 12 carbon atoms, and may be substituted with methyl or the like. Examples of such an aryl group include a tolyl group and a mesityl group.

Specific examples of the amine salt and the amine compound include 1-aminopyridinium iodide, tetrabutylammonium azide, hydroxylamine-O-sulfonic acid, 1-aminopyridinium mesitylene sulfonate, O-(mesitylenesulfonyl)hydroxylamine, and 1,1,1-trimethylhydrazinium trifluoroacetate.

When a nucleophile is used in each of the above reactions, examples of the nucleophile include tertiary amines such as triethylamine, quinuclidine and 1,4-diazabicyclo[2.2.2]octane, imidazole derivatives such as 1-methylimidazole, and pyridine derivatives such as pyridine and 4-dimethylaminopyridine.

When a base is used in each of the above reactions, examples of the base include inorganic compounds such as sodium hydroxide, potassium hydroxide, calcium hydroxide, sodium carbonate, potassium carbonate, sodium fluoride, and potassium fluoride; organic nitrogen derivatives such as pyridine, triethylamine, diisopropylethylamine, diazabicyclononene, diazabicycloundecene, methyltriazabicyclodecene, and diazabicyclooctane; and phosphorus derivatives such as phosphazene bases.

When an alcohol (ROH) is used in each of the above reactions, the type of alcohol is not particularly limited as long as an ester of the alcohol and 3,3,3-trifluoro-2-(trifluoromethyl)propanoate can be formed, and the alcohol can be appropriately used according to the desired structure of isoxazole.

When hydrogen fluoride (HF) is generated in the method for producing a fluorine-containing isoxazole compound in the present embodiment, for example, a halogenated hydrogen scavenger may be used. By using the halogenated hydrogen scavenger, the step of recovering hydrogen fluoride can be omitted, and the fluorine-containing isoxazole compound represented by the above General Formula (A) can be obtained more easily.

In the reaction of the above (a), a cyclic isoxazole structure is formed between the fluorine-containing carbonyl compound represented by General Formula (6) and the amino group of hydroxylamine represented by General Formula (7). X and CF₃ of the fluorine-containing carbonyl compound represented by General Formula (6) are respectively positioned at the 3-position and 4-position of the formed isoxazole structure, and OH formed due to ring structure formation is positioned at the 5-position of the isoxazole structure.

In the reaction of the above (b), a cyclic isoxazole structure is formed between the fluorine-containing carbonyl compound represented by General Formula (8) and the amino group of hydroxylamine represented by General Formula (7), the compound represented by General Formula (5) is substituted at the 3-position of the isoxazole structure, and OH formed due to ring structure formation is positioned at the 5-position of the isoxazole structure. CF₃ of the fluorine-containing carbonyl compound represented by General Formula (8) is positioned at the 4-position of the formed isoxazole structure.

In the reaction of the above (c), a cyclic isoxazole structure is formed between the reaction product, which is obtained by reacting, in the presence of a base, the fluorine-containing carbonyl compound represented by General Formula (9) with the obtained reaction product represented by General Formula (5), and the amino group of hydroxylamine represented by General Formula (7), the compound represented by General Formula (5) is substituted at the 3-position of the isoxazole structure, and OH formed due to ring structure formation is positioned at the 5-position of the isoxazole structure. CF₃ of the fluorine-containing carbonyl compound represented by General Formula (9) is positioned at the 4-position of the formed isoxazole structure.

In the reaction of the above (d), a cyclic isoxazole structure is formed between the reaction product, which is obtained by reacting, in the presence of a base, the intermediate reactant - which is obtained by reacting the fluorine-containing carbonyl compound represented by General Formula (10) with an alcohol - with the compound represented by General Formula (5), and the amino group of hydroxylamine represented by General Formula (7), the compound represented by General Formula (5) is substituted at the 3-position of the isoxazole structure, and OH formed due to ring structure formation is positioned at the 5-position of the isoxazole structure. CF₃ of the fluorine-containing carbonyl compound represented by General Formula (10) is positioned at the 4-position of the formed isoxazole structure.

In the reaction of the above (e), a cyclic isoxazole structure is formed between the reaction product, which is obtained by reacting, in the presence of a base, the intermediate reactant - which is obtained by carbonylating, in the presence of a nucleophile, the fluoroisobutene derivative represented by General Formula (11) and further reacting the fluoroisobutene derivative with an alcohol - with the compound represented by General Formula (5), and the amino group of hydroxylamine represented by General Formula (7), the compound represented by General Formula (5) is substituted at the 3-position of the isoxazole structure, and OH formed due to ring structure formation is positioned at the 5-position of the isoxazole structure. CF₃ of the fluoroisobutene derivative represented by General Formula (11) is positioned at the 4-position of the formed isoxazole structure.

In the reaction of the above (f), a cyclic isoxazole structure is formed between the reaction product, which is obtained by reacting, in the presence of a base, the intermediate reactant - which is obtained by carbonylating, in the presence of a nucleophile, the compound obtained by an elimination reaction of the fluoroisobutane derivative represented by General Formula (12) and further reacting the compound with an alcohol - with the compound represented by General Formula (5), and the amino group of hydroxylamine represented by General Formula (7), the compound represented by General Formula (5) is substituted at the 3-position of the isoxazole structure, and OH formed due to ring structure formation is positioned at the 5-position of the isoxazole structure. CF₃ of the fluorine-containing carbonyl compound represented by General Formula (12) is positioned at the 4-position of the formed isoxazole structure.

In the reaction of the above (g), a cyclic isoxazole structure is formed between the fluorine-containing carbonyl compound represented by General Formula (13) and the amino group of the amine salt or the amine compound. X, CF₃, and OR of the fluorine-containing carbonyl compound represented by General Formula (13) are respectively positioned at the 3-position, 4-position, and 5-position of the formed isoxazole structure.

In the reaction of the above (h), a cyclic isoxazole structure is formed between the fluorine-containing carbonyl compound represented by General Formula (14) and the amino group of the amine salt or the amine compound. CF₃ and OR of the fluorine-containing carbonyl compound represented by General Formula (14) are respectively positioned at the 4-position and 5-position of the formed isoxazole structure. When the fluorine-containing carbonyl compound represented by General Formula (14) is further reacted with the compound represented by General Formula (5), the compound represented by General Formula (5) is substituted at the 3-position of the isoxazole structure, and when the compound represented by General Formula (5) is not used, F of the fluorine-containing carbonyl compound represented by General Formula (14) is positioned at the 3-position of the isoxazole structure.

In the reaction of the above (i), a cyclic isoxazole structure is formed between the reaction product, which is obtained by reacting, in the presence of a base, the fluorine-containing carbonyl compound represented by General Formula (15) with the compound represented by General Formula (5), and the amino group of the amine salt or the amine compound, and the compound represented by General Formula (5) is substituted at the 3-position of the isoxazole structure. CF₃ and OR of the fluorine-containing carbonyl compound represented by General Formula (15) are respectively positioned at the 4-position and 5-position of the formed isoxazole structure.

In the reaction of the above (j), a cyclic isoxazole structure is formed between the reaction product, which is obtained by reacting, in the presence of a base, the intermediate reactant - which is obtained by reacting the fluorine-containing carbonyl compound represented by General Formula (10) with an alcohol - with the compound represented by General Formula (5), and the amino group of the amine salt or the amine compound, and the compound represented by General Formula (5) is substituted at the 3-position of the isoxazole structure. CF₃ of the fluorine-containing carbonyl compound represented by General Formula (10) is positioned at the 4-position of the formed isoxazole structure. At the 5-position of the isoxazole structure, OR of an intermediate reactant obtained by reaction of a fluorine-containing carbonyl compound represented by General Formula (10) with an alcohol (ROH) is positioned.

In the reaction of the above (k), a cyclic isoxazole structure is formed between the reaction product, which is obtained by reacting, in the presence of a base, the intermediate reactant - which is obtained by carbonylating, in the presence of a nucleophile, the fluoroisobutene derivative represented by General Formula (11) and further reacting the fluoroisobutene derivative with an alcohol - with the compound represented by General Formula (5), and the amino group of the amine salt or the amine compound, and the compound represented by General Formula (5) is substituted at the 3-position of the isoxazole structure. CF₃ of the fluorine-containing carbonyl compound represented by General Formula (11) is positioned at the 4-position of the formed isoxazole structure. The OR of an intermediate reactant, which is obtained by reacting a compound obtained by carbonylating a fluoroisobutene derivative represented by General Formula (11) with an alcohol (ROH) is positioned at the 5-position of the isoxazole structure.

In the reaction of the above (l), a cyclic isoxazole structure is formed between the reaction product, which is obtained by reacting, in the presence of a base, the intermediate reactant - which is obtained by carbonylating, in the presence of a nucleophile, the compound obtained by an elimination reaction of a fluoroisobutane derivative represented by General Formula (12) with an alcohol - with the compound represented by General Formula (5), and the amino group of the amine salt or the amine compound, and the compound represented by General Formula (5) is substituted at the 3-position of the isoxazole structure. CF₃ of the fluorine-containing carbonyl compound represented by General Formula (12) is positioned at the 4-position of the formed isoxazole structure. At the 5-position of the isoxazole structure, OR of an intermediate reactant obtained by reacting a compound obtained by performing an elimination reaction of the fluoroisobutene derivative represented by General Formula (12) and further carbonylating the fluoroisobutene derivative with an alcohol (ROH) is positioned.

In the reaction of the above (m), a cyclic isoxazole structure is formed between the fluorine-containing carbonyl compound represented by General Formula (10) and the amino group of hydroxylamine represented by General Formula (7). The compound represented by General Formula (5) is substituted at the 3-position of the isoxazole structure, and OH formed due to ring structure formation is positioned at the 5-position of the isoxazole structure. CF₃ of the fluorine-containing carbonyl compound represented by General Formula (10) is positioned at the 4-position of the formed isoxazole structure.

In the reaction of the above (n), a cyclic isoxazole structure is formed between the reaction product, which is obtained by reacting, in the presence of a base, the intermediate reactant - which is obtained by carbonylating, in the presence of a nucleophile, the fluoroisobutene derivative represented by General Formula (11) and further reacting the fluoroisobutene derivative with an alcohol - with the compound represented by General Formula (5), and the amino group of hydroxylamine represented by General Formula (7). The compound represented by General Formula (5) is substituted at the 3-position of the isoxazole structure, and OH formed due to ring structure formation is positioned at the 5-position of the isoxazole structure. CF₃ of the fluoroisobutene derivative represented by General Formula (11) is positioned at the 4-position of the formed isoxazole structure.

In the reaction of the above (o), a cyclic isoxazole structure is formed between the reaction product, which is obtained by reacting, in the presence of a base, the intermediate reactant - which is obtained by carbonylating, in the presence of a nucleophile, the compound obtained by an elimination reaction of the fluoroisobutane derivative represented by General Formula (12) with an alcohol - with the compound represented by General Formula (5), and the amino group of hydroxylamine represented by General Formula (7). The compound represented by General Formula (5) is substituted at the 3-position of the isoxazole structure, and OH formed due to ring structure formation is positioned at the 5-position of the isoxazole structure. CF₃ of the fluoroisobutene derivative represented by General Formula (12) is positioned at the 4-position of the formed isoxazole structure.

In the reaction of the above (p), a cyclic isoxazole structure is formed between the fluoroisobutylene derivative represented by General Formula (11) and the amino group of hydroxylamine represented by General Formula (7), and further, the compound represented by General Formula (5) is substituted on the isoxazole structure. -OR' and CF₃ of the fluoroisobutylene derivative are positioned at the 3-position and 4-position of the formed isoxazole structure, respectively, and X of the compound represented by General Formula (5) is positioned at the 5-position of the isoxazole structure.

In the reaction of the above (q), a cyclic isoxazole structure is formed between the fluoroisobutylene derivative represented by General Formula (11) and the amino group of hydroxylamine represented by General Formula (7). - OR', CF₃, and F of the fluoroisobutylene derivative are positioned at the 3-position, 4-position and 5-position of the formed isoxazole structure, respectively.

In the reaction of the above (r), a cyclic isoxazole structure is formed between the fluoroisobutane derivative represented by General Formula (12) and the amino group of hydroxylamine represented by General Formula (7), and further, the compound represented by General Formula (5) is substituted on the isoxazole structure. -OR' and CF₃ of the fluoroisobutane derivative are positioned at the 3-position and 4-position of the formed isoxazole structure, respectively, and X of the compound represented by General Formula (5) is positioned at the 5-position of the isoxazole structure.

In the reaction of the above (s), a cyclic isoxazole structure is formed between the fluoroisobutane derivative represented by General Formula (12) and the amino group of hydroxylamine represented by General Formula (7). -OR', CF₃, and F of the fluoroisobutane derivative are positioned at the 3-position, 4-position and 5-position of the formed isoxazole structure, respectively.

The halogenated hydrogen scavenger is a substance having a function of capturing generated hydrogen fluoride (HF). Examples of the halogenated hydrogen scavengers include inorganic compounds such as sodium hydroxide, potassium hydroxide, calcium hydroxide, sodium carbonate, potassium carbonate, sodium fluoride, and potassium fluoride; organic nitrogen derivatives such as pyridine, triethylamine, diisopropylethylamine, diazabicyclononene, diazabicycloundecene, methyltriazabicyclodecene, and diazabicyclooctane; and phosphorus derivatives such as phosphazene bases.

Each reaction of the above (a) to (s) may be performed in the presence of a fluoride ion scavenger as necessary. The fluoride ion scavenger is preferably a salt of a cation of lithium, sodium, magnesium, potassium, calcium, or tetramethylammonium; and an anion of trifluoroacetic acid, heptafluorobutyric acid, methanesulfonic acid, ethanesulfonic acid, benzenesulfonic acid, toluenesulfonic acid, trifluoromethanesulfonic acid, nonafluorobutanesulfonic acid, bis(trifluoromethanesulfonyl)imide, bis(nonafluorobutanesulfonyl)imide, N,N-hexafluoropropane-1,3-disulfonylimide, tetraphenylboric acid, tetrakis[3,5-bis(trifluoromethyl)phenyl]borate, or tetrakis(pentafluorophenyl)borate. Among them, a potassium salt or a sodium salt is preferably used, and a sodium salt is more preferably used. It is considered that the cation derived from the fluoride ion scavenger captures the fluorine ion released during the reaction and is precipitated as a salt having low solubility in an organic solvent to promote the reaction, whereby a fluorine-containing isoxazole compound can be obtained in high yield.

The reaction temperature in the reactions of the above (a) to (s) is preferably 0 to 100°C, more preferably 5 to 50°C, and still more preferably 10 to 20°C. The reaction time in the reactions of the above (a) to (s) is preferably 0.5 to 48 hours, more preferably 1 to 36 hours, and still more preferably 2 to 12 hours.

The solvent used in the reactions of the above (a) to (s) is preferably an organic solvent, and examples thereof include aprotic polar solvents such as tetrahydrofuran, monoglyme, diglyme, triglyme, tetraglyme, acetonitrile, dimethylformamide, dimethylacetamide, methylpyrrolidone, 4-methyltetrahydropyran, dimethylethyleneurea, tetramethylurea, dimethylsulfoxide, and sulfolane; and biphasic solvents of a protic polar solvent such as methanol and water and a non-water-soluble solvent such as dichloromethane, toluene, and diethyl ether. As a catalyst for the reactions of the above (a) to (s), a quaternary ammonium halide such as benzyltriethylammonium chloride, a quaternary phosphonium halide, a crown ether, or the like can be used optionally.

Based on the above embodiments, the present invention relates to the following [1] to [25].
[1] A fluorine-containing isoxazole compound represented by the following General Formula (A). 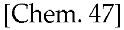 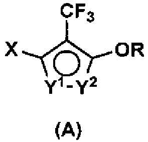 (In the above General Formula (A),
   R represents a hydrogen atom or a substituted or unsubstituted hydrocarbon group having 1 to 12 carbon atoms,
   X represents a halogen atom, -OA¹, -O-NA¹A², -NA¹A², -NA¹(OA²), -NA³-NA¹A², -NA⁴, or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   Y¹ and Y² each independently represent N or O, and are different from each other.)
[2] The fluorine-containing isoxazole compound according to the above [1], in which the fluorine-containing isoxazole compound is a compound represented by the following General Formula (1). 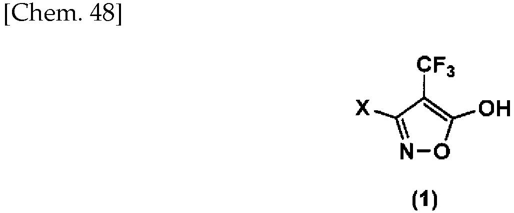 (In the above General Formula (1),
   X represents a halogen atom, -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ, and
   n is an integer of 1 to 20.)
[3] The fluorine-containing isoxazole compound according to the above [1], in which the fluorine-containing isoxazole compound is a compound represented by the following General Formula (2). 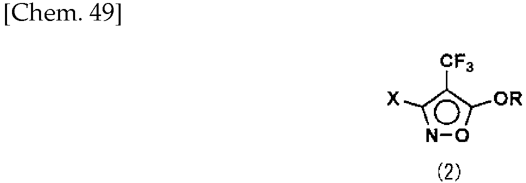 (In the above General Formula (2),
   R represents a substituted or unsubstituted hydrocarbon having 1 to 12 carbon atoms,
   X represents a halogen atom, -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ, and
   n is an integer of 1 to 20.)
[4] The fluorine-containing isoxazole compound according to the above [1], in which the fluorine-containing isoxazole compound is a compound represented by the following General Formula (3). 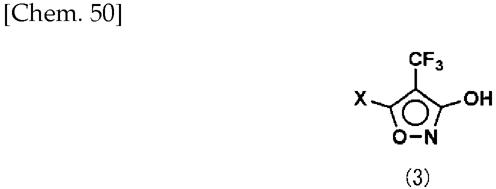 (In the above General Formula (3),
   X represents a halogen atom, -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ, and
   n is an integer of 1 to 20.)
[5] The fluorine-containing isoxazole compound according to the above [1], in which the fluorine-containing isoxazole compound is a compound represented by the following General Formula (4). 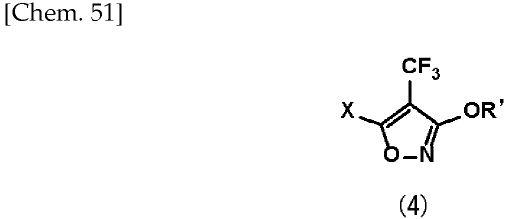 (In the above General Formula (4),
   R' represents a substituted or unsubstituted hydrocarbon having 1 to 12 carbon atoms,
   X represents a halogen atom, -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ, and
   n is an integer of 1 to 20.)
[6] A method for producing a fluorine-containing isoxazole compound according to the present embodiment includes a step of synthesizing a fluorine-containing isoxazole compound represented by the following General Formula (A)
   (I) by reacting a fluorine-containing compound selected from a fluorine-containing carbonyl compound, a fluoroisobutene derivative, and a fluoroisobutane derivative, hydroxylamine or a salt thereof, and optionally a compound represented by the following General Formula (5) or a salt thereof, or
   (II) by reacting a fluorine-containing compound selected from a fluorine-containing carbonyl compound, a fluoroisobutene derivative, and a fluoroisobutane derivative, an amine salt or an amine compound, and optionally a compound represented by the following General Formula (5) or a salt thereof. 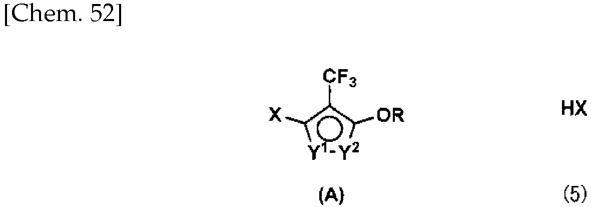 (In the above General Formulae (A) and (5),
      R represents a hydrogen atom or a substituted or unsubstituted hydrocarbon group having 1 to 12 carbon atoms,
      X represents a halogen atom, -OA¹, -O-NA¹A², -NA¹A², -NA¹(OA²), -NA³-NA¹A², -NA⁴, or a heterocycle,
      A¹, A², and A³ each independently represent a substituted or unsubstituted hydrocarbon group having 1 to 12 carbon atoms or a heterocycle,
      A⁴ represents an alkylene group represented by =CₙH₂ₙ,
      n is an integer of 1 to 20, and
      Y¹ and Y² each independently represent N or O, and are different from each other.)
[7] The method for producing a fluorine-containing isoxazole compound according to the above [6], the method including a step of obtaining a fluorine-containing isoxazole compound represented by the following General Formula (1) by reacting a fluorine-containing carbonyl compound represented by the following General Formula (6) with hydroxylamine represented by the following General Formula (7) or a salt thereof. 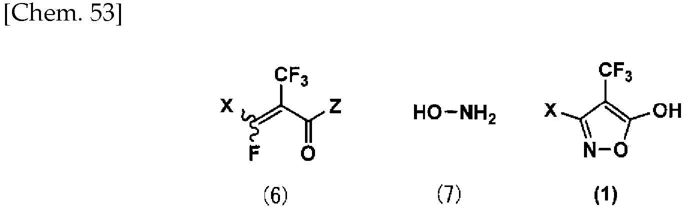 (In the above General Formulae (1), (6), and (7),
   X represents a halogen atom, -OA¹, -O-NA¹A², -NA¹A², -NA^{I}(OA²), -NA³-NA¹A², -NA⁴, or a heterocycle,
   Z represents -OA³, -O-NA⁵A⁶, -NA⁵A⁶, -NA⁵(OA⁶), or -NA⁷-NA⁵A⁶,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20,
   A⁵, A⁶, and A⁷ each independently represent a substituted or unsubstituted hydrocarbon group having 1 to 12 carbon atoms, and
   a wavy bond indicates a single bond representing stereoisomerism of (E) or (Z).)
[8] The method for producing a fluorine-containing isoxazole compound according to the above [6], the method including a step of obtaining a fluorine-containing isoxazole compound represented by the following General Formula (1) by reacting a fluorine-containing carbonyl compound represented by the following General Formula (8), a compound represented by the following General Formula (5) or a salt thereof, and hydroxylamine represented by the following General Formula (7) or a salt thereof. 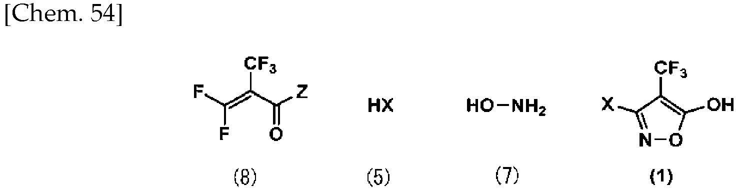 (In the above General Formulae (1), (5), and (8),
   X represents a halogen atom, -OA¹, -O-NA¹A², -NA¹A², -NA¹(OA²), -NA³-NA¹A², -NA⁴, or a heterocycle,
   Z represents -OA⁵ , -O-NA⁶A⁷, -NA⁵A⁶, -NA⁵(OA⁶), or -NA⁷-NA⁵A⁶, and A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   A⁵, A⁶, and A⁷ each independently represent a substituted or unsubstituted hydrocarbon group having 1 to 12 carbon atoms.)
[9] The method for producing a fluorine-containing isoxazole compound according to the above [6], including a step of obtaining a fluorine-containing isoxazole compound represented by the following General Formula (1) by reacting a fluorine-containing carbonyl compound represented by the following General Formula (9) with a compound represented by the following General Formula (5) or a salt thereof in the presence of a base, and then further reacting the obtained reaction product with hydroxylamine represented by the following General Formula (7) or a salt thereof. 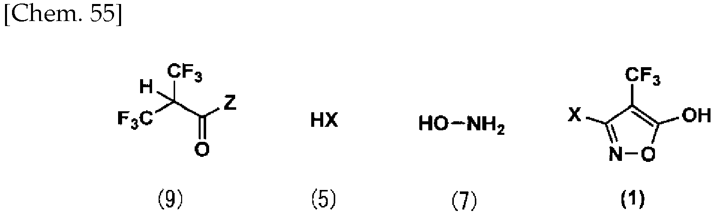 (In the above General Formulae (1), (5), and (9),
   X represents a halogen atom, -OA¹, -O-NA¹A², -NA¹A², -NA^{I}(OA²), -NA³-NA¹A², -NA⁴, or a heterocycle,
   Z represents -OA³, -O-NA⁶A⁷, -NA⁵A⁶, -NA⁵(OA⁶), or -NA⁷-NA⁵A⁶,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   A⁵, A⁶, and A⁷ each independently represent a substituted or unsubstituted hydrocarbon group having 1 to 12 carbon atoms.)
[10] The method for producing a fluorine-containing isoxazole compound according to the above [6], the method including a step of obtaining a fluorine-containing isoxazole compound represented by the following General Formula (1) by reacting a fluorine-containing carbonyl compound represented by the following General Formula (10) with an alcohol, then reacting the obtained reaction product with a compound represented by the following General Formula (5) or a salt thereof in the presence of a base, and then reacting the reaction product with hydroxylamine represented by the following General Formula (7) or a salt thereof. 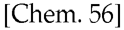 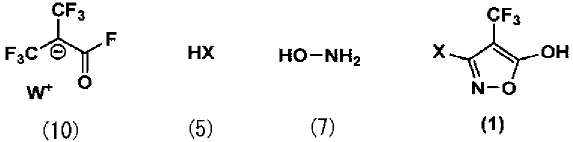 (In the above General Formulae (1), (5), and (10),
   X represents a halogen atom, -OA¹, -O-NA¹A², -NA¹A², -NA^{I}(OA²), -NA³-NA¹A², -NA⁴, or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   W⁺ represents an ammonium cation, an imidazolium cation, a pyridinium cation, a quinuclidinium cation, or a phosphonium cation.)
[11] The method for producing a fluorine-containing isoxazole compound according to the above [6], the method including a step of obtaining a fluorine-containing isoxazole compound represented by the following General Formula (1) by carbonylating a fluoroisobutene derivative represented by the following General Formula (11) in the presence of a nucleophile, further reacting the fluoroisobutene derivative with an alcohol, then reacting the obtained reaction product with a compound represented by the following General Formula (5) or a salt thereof in the presence of a base, and then reacting the reaction product with hydroxylamine represented by the following General Formula (7) or a salt thereof. 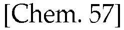 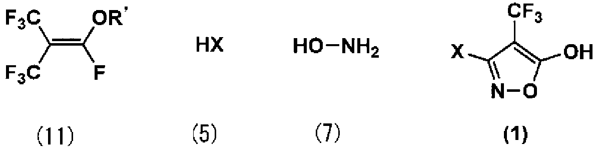 (In the above General Formulae (1), (5), and (11),
   R' represents a substituted or unsubstituted hydrocarbon group having 1 to 12 carbon atoms,
   X represents a halogen atom, -OA¹, O-NA¹A², -NA¹(OA²), -NA¹A², -NA³-NA¹A², -NA⁴ or a heterocycle,
   A¹, A², and A³ each independently represent a substituted or unsubstituted hydrocarbon group having 1 to 12 carbon atoms,
   A⁴ represents an alkylene group represented by =CₙH₂ₙ, and
   n is an integer of 1 to 20.)
[12] The method for producing a fluorine-containing isoxazole compound according to the above [6], the method including a step of obtaining a fluorine-containing isoxazole compound represented by the following General Formula (1) by carbonylating a compound obtained by a elimination reaction of the fluoroisobutane derivative following General Formula (12) in the presence of a nucleophile, further reacting the fluoroisobutene derivative with an alcohol, then reacting the obtained reaction product with a compound represented by the following General Formula (5) or a salt thereof in the presence of a base, and then reacting the reaction product with hydroxylamine represented by the following General Formula (7) or a salt thereof. 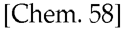 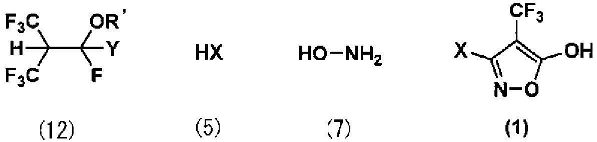 (In the above General Formulae (1), (5), and (12),
   R' represents a substituted or unsubstituted hydrocarbon group having 1 to 12 carbon atoms,
   X represents a halogen atom, -OA¹, -O-NA¹A², -NA¹A², -NA^{I}(OA²), -NA³-NA¹A², -NA⁴, or a heterocycle,
   Y represents a halogen atom, -OB¹, -SOₘB¹, or -NB¹B²,
   m is an integer of 0 to 3,
   A¹, A², and A³ each independently represent a substituted or unsubstituted hydrocarbon group having 1 to 12 carbon atoms,
   A⁴ represents an alkylene group represented by =CₙH₂ₙ,
   n is an integer of 1 to 20, and
   B¹ and B² each independently represent a substituted or unsubstituted hydrocarbon group having 1 to 12 carbon atoms.)
[13] The method for producing a fluorine-containing isoxazole compound according to the above [6], the method including a step of obtaining a fluorine-containing isoxazole compound represented by the following General Formula (2) by reacting a fluorine-containing carbonyl compound represented by the following General Formula (13) with an amine salt or an amine compound. 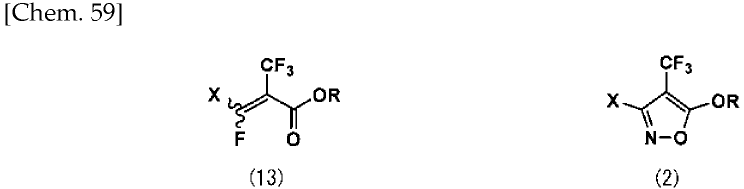 (In the above General Formulae (2) and (13),
   R represents a substituted or unsubstituted hydrocarbon group having 1 to 12 carbon atoms,
   X represents -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   a wavy bond indicates a single bond representing stereoisomerism of (E) or (Z).)
[14] The method for producing a fluorine-containing isoxazole compound according to the above [6], the method including a step of obtaining a fluorine-containing isoxazole compound represented by the following General Formula (2) by reacting the fluorine-containing carbonyl compound represented by the following General Formula (14) with an amine salt or an amine compound, and optionally with a compound represented by the following General Formula (5) or a salt thereof. 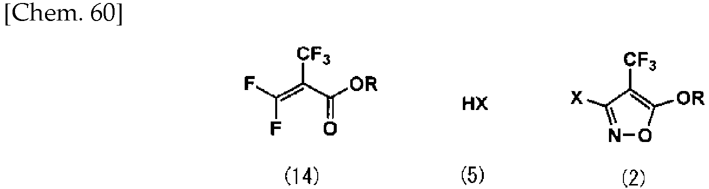 (In the above General Formulae (2), (5), and (14),
   R represents a substituted or unsubstituted hydrocarbon group having 1 to 12 carbon atoms,
   X represents a halogen atom, -OA¹, O-NA¹A², -NA^{I}(OA²), -NA¹A², -NA³-NA¹A², -NA⁴ or a heterocycle,
   A¹, A², and A³ each independently represent a substituted or unsubstituted hydrocarbon group having 1 to 12 carbon atoms,
   A⁴ represents an alkylene group represented by =CₙH₂ₙ, and
   n is an integer of 1 to 20.)
[15] The method for producing a fluorine-containing isoxazole compound according to the above [6], including a step of obtaining a fluorine-containing isoxazole compound represented by the following General Formula (2) by reacting a fluorine-containing carbonyl compound represented by the following General Formula (15) with a compound represented by the following General Formula (5) or a salt thereof in the presence of a base, and then reacting the obtained reaction product with an amine salt or an amine compound. 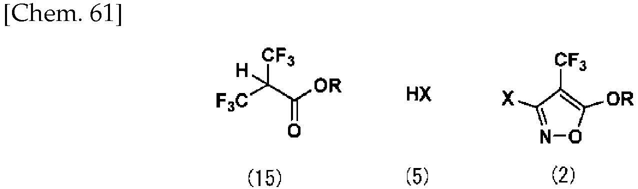 (In the above General Formulae (2), (5), and (15),
   R represents a substituted or unsubstituted hydrocarbon group having 1 to 12 carbon atoms,
   X represents a halogen atom, -OA¹, O-NA¹A², -NA¹A², -NA¹(OA²), -NA³-NA¹A², -NA⁴ or a heterocycle,
   A¹, A², and A³ each independently represent a hydrogen atom and a substituted or unsubstituted hydrocarbon group having 1 to 12 carbon atoms or a heterocycle,
   A⁴ represents an alkylene group represented by =CₙH₂ₙ, and
   n is an integer of 1 to 20.)
[16] The method for producing a fluorine-containing isoxazole compound according to the above [6], the method including a step of obtaining a fluorine-containing isoxazole compound represented by the following General Formula (2) by reacting a fluorine-containing carbonyl compound represented by the following General Formula (10) with an alcohol, then reacting the obtained reaction product with a compound represented by the following General Formula (5) or a salt thereof in the presence of a base, and then reacting the reaction product with an amine salt or an amine compound. 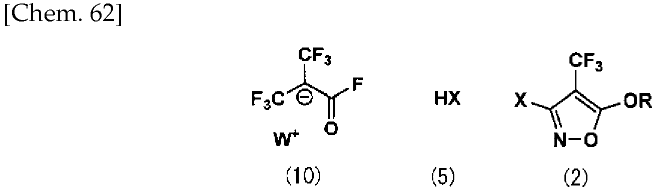 (In the above General Formulae (2), (5), and (10),
   R represents a substituted or unsubstituted hydrocarbon group having 1 to 12 carbon atoms,
   X represents a halogen atom, -OA¹, -O-NA¹A², -NA¹A², -NA^{I}(OA²), -NA³-NA¹A², -NA⁴, or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   W⁺ represents an ammonium cation, an imidazolium cation, a pyridinium cation, a quinuclidinium cation, or a phosphonium cation.)
[17] The method for producing a fluorine-containing isoxazole compound according to the above [6], the method including a step of obtaining a fluorine-containing isoxazole compound represented by the following General Formula (2) by carbonylating a fluoroisobutene derivative represented by the following General Formula (11) in the presence of a nucleophile, further reacting the fluoroisobutene derivative with an alcohol, then reacting the obtained reaction product with a compound represented by the following General Formula (5) or a salt thereof in the presence of a base, and then reacting the reaction product with an amine salt or an amine compound. 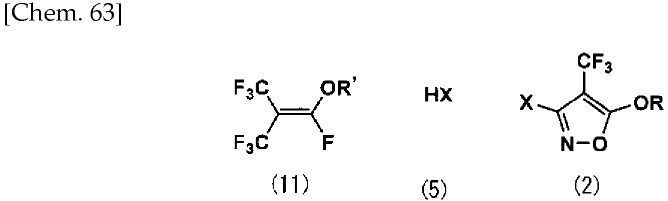 (In the above General Formulae (2), (5), and (11),
   R and R' each independently represent a substituted or unsubstituted hydrocarbon having 1 to 12 carbon atoms,
   X represents a halogen atom, -OA¹, O-NA¹A², -NA¹A², -NA^{I}(OA²), -NA³-NA¹A², -NA⁴ or a heterocycle,
   A¹, A², and A³ each independently represent a substituted or unsubstituted hydrocarbon group having 1 to 12 carbon atoms,
   A⁴ represents an alkylene group represented by =CₙH₂ₙ, and
   n is an integer of 1 to 20.)
[18] The method for producing a fluorine-containing isoxazole compound according to the above [6], the method including a step of obtaining a fluorine-containing isoxazole compound represented by the following General Formula (2) by carbonylating a compound obtained by an elimination reaction of the fluoroisobutane derivative represented by following General Formula (12) in the presence of a nucleophile, further reacting the compound with an alcohol, then reacting the obtained reaction product with the compound represented by the following General Formula (5) or a salt thereof in the presence of a base, and then reacting the reaction product with an amine salt or an amine compound. 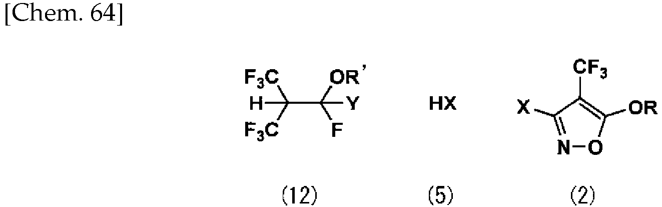 (In the above General Formulae (2), (5), and (12),
   R and R' each independently represent a substituted or unsubstituted hydrocarbon group having 1 to 12 carbon atoms,
   X represents a halogen atom, -OA¹, -O-NA¹A², -NA¹A², -NA^{I}(OA²), -NA³-NA¹A²-NA⁴, or a heterocycle,
   Y represents a halogen atom, -OB¹, -SOₘB¹, or -NB¹B²,
   m is an integer of 0 to 3,
   A¹, A², and A³ each independently represent a substituted or unsubstituted hydrocarbon group having 1 to 12 carbon atoms,
   A⁴ represents an alkylene group represented by =CₙH₂ₙ,
   n is an integer of 1 to 20, and
   B¹ and B² each independently represent a substituted or unsubstituted hydrocarbon group having 1 to 12 carbon atoms.)
[19] The method for producing a fluorine-containing isoxazole compound according to the above [6], the method including a step of obtaining a fluorine-containing isoxazole compound represented by the following General Formula (3) by reacting a fluorine-containing carbonyl compound represented by the following General Formula (10) with hydroxylamine represented by the following General Formula (7) or a salt thereof, and further reacting the obtained reaction product with a compound represented by the following General Formula (5) or a salt thereof. 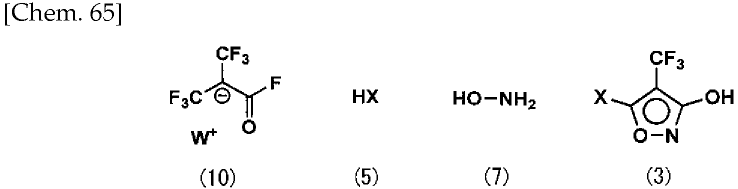 (In the above General Formulae (3), (5), and (10),
   X represents a halogen atom, -OA¹, -O-NA¹A², -NA¹A², -NA¹(OA²), -NA³-NA¹A², -NA⁴, or a heterocycle,
   A¹, A², and A³ each independently represent a substituted or unsubstituted hydrocarbon group having 1 to 12 carbon atoms or a heterocycle,
   A⁴ represents an alkylene group represented by =CₙH₂ₙ,
   n is an integer of 1 to 20, and
   W⁺ represents an ammonium cation, an imidazolium cation, a pyridinium cation, a quinuclidinium cation, or a phosphonium cation.)
[20] The method for producing a fluorine-containing isoxazole compound according to the above [6], the method including a step of obtaining a fluorine-containing isoxazole compound represented by the following General Formula (3) by carbonylating a fluoroisobutene derivative represented by the following General Formula (11) in the presence of a nucleophile, further reacting the fluoroisobutene derivative with hydroxylamine represented by the following General Formula (7) or a salt thereof, and then further reacting the obtained reaction product with a compound represented by the following General Formula (5) or a salt thereof. 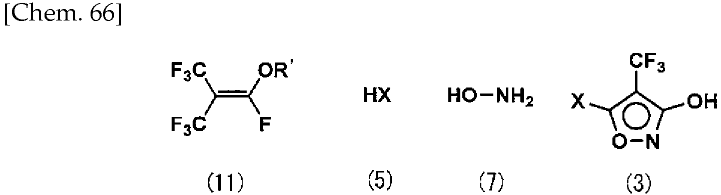 (In the above General Formulae (3), (5), and (11),
   R' represents a substituted or unsubstituted hydrocarbon having 1 to 12 carbon atoms,
   X represents a halogen atom, -OA¹, O-NA¹A², -NA¹A², -NA¹(OA²), -NA³-NA¹A², -NA⁴ or a heterocycle,
   A¹, A², and A³ each independently represent a substituted or unsubstituted hydrocarbon group having 1 to 12 carbon atoms,
   A⁴ represents an alkylene group represented by =CₙH₂ₙ, and
   n is an integer of 1 to 20.)
[21] The method for producing a fluorine-containing isoxazole compound according to the above [6], the method including a step of obtaining a fluorine-containing isoxazole compound represented by the following General Formula (3) by carbonylating a fluoroisobutane derivative represented by the following General Formula (12) in the presence of a nucleophile, further reacting the fluoroisobutane derivative with hydroxylamine represented by the following General Formula (7) or a salt thereof, and then reacting the obtained reaction product with a compound represented by the following General Formula (5) or a salt thereof. 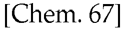 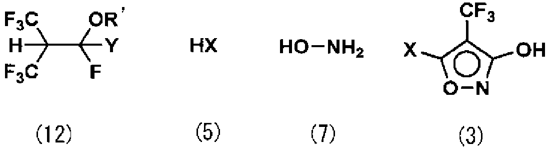 (In the above General Formulae (3), (5), and (12),
   R' represents a substituted or unsubstituted hydrocarbon group having 1 to 12 carbon atoms,
   X represents a halogen atom, -OA¹, -O-NA¹A², -NA¹A², -NA^{I}(OA²), -NA³-NA¹A²-NA⁴, or a heterocycle,
   Y represents a halogen atom, -OB¹, -SOₘB¹, or -NB¹B²,
   m is an integer of 0 to 3,
   A¹, A², and A³ each independently represent a substituted or unsubstituted hydrocarbon group having 1 to 12 carbon atoms,
   A⁴ represents an alkylene group represented by =CₙH₂ₙ,
   n is an integer of 1 to 20, and
   B¹ and B² each independently represent a substituted or unsubstituted hydrocarbon group having 1 to 12 carbon atoms.)
[22] The method for producing a fluorine-containing isoxazole compound according to the above [6], the method including a step of obtaining a fluorine-containing isoxazole compound represented by the following General Formula (4) by reacting a fluoroisobutene derivative represented by the following General Formula (11), a compound represented by the following General Formula (5) or a salt thereof, and hydroxylamine represented by the following General Formula (7) or a salt thereof. 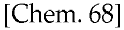 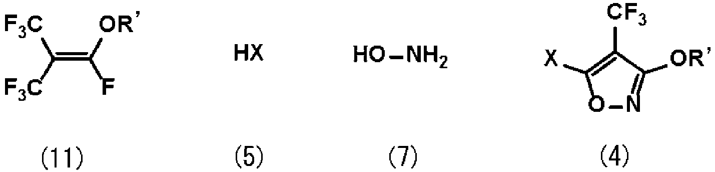 (In the above General Formulae (4), (5), and (11),
   R' represents a substituted or unsubstituted hydrocarbon having 1 to 12 carbon atoms,
   X represents a halogen atom, -OA¹, O-NA¹A², -NA¹(OA²), -NA¹A², -NA³-NA¹A², -NA⁴ or a heterocycle,
   A¹, A², and A³ each independently represent a substituted or unsubstituted hydrocarbon group having 1 to 12 carbon atoms,
   A⁴ represents an alkylene group represented by =CₙH₂ₙ, and
   n is an integer of 1 to 20.)
[23] The method for producing a fluorine-containing isoxazole compound according to the above [6], the method including a step of obtaining a fluorine-containing isoxazole compound represented by the following General Formula (4-1) by reacting a fluoroisobutene derivative represented by the following General Formula (11) with hydroxylamine represented by the following General Formula (7) or a salt thereof. 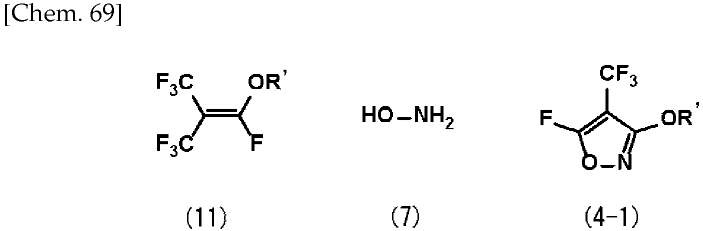 (In the above General Formulae (4-1) and (11),
   R' represents a substituted or unsubstituted hydrocarbon group having 1 to 12 carbon atoms.)
[24] The method for producing a fluorine-containing isoxazole compound according to the above [6], the method including a step of obtaining a fluorine-containing isoxazole compound represented by the following General Formula (4) by reacting a fluoroisobutane derivative represented by the following General Formula (12), a compound represented by the following General Formula (5) or a salt thereof, and hydroxylamine represented by the following General Formula (7) or a salt thereof. 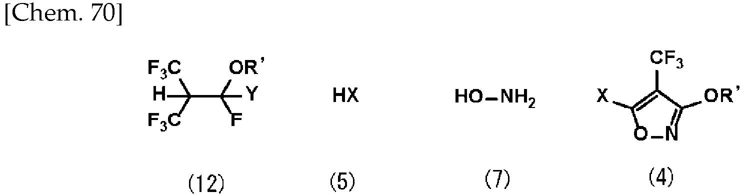 (In the above General Formulae (4), (5), and (12),
   R' represents a substituted or unsubstituted hydrocarbon group having 1 to 12 carbon atoms,
   X represents a halogen atom, -OA¹, -O-NA¹A², -NA¹A², -NA^{I}(OA²), -NA³-NA¹A², -NA⁴, or a heterocycle,
   Y represents a halogen atom, -OB¹, -SOₘB¹, or -NB¹B²,
   m is an integer of 0 to 3,
   A¹, A², and A³ each independently represent a substituted or unsubstituted hydrocarbon group having 1 to 12 carbon atoms,
   A⁴ represents an alkylene group represented by =CₙH₂ₙ,
   n is an integer of 1 to 20, and
   B¹ and B² each independently represent a substituted or unsubstituted hydrocarbon group having 1 to 12 carbon atoms.)
[25] The method for producing a fluorine-containing isoxazole compound according to the above [6], the method including a step of obtaining a fluorine-containing isoxazole compound represented by the following General Formula (4-1) by reacting a fluoroisobutane derivative represented by the following General Formula (12) with hydroxylamine represented by the following General Formula (7). 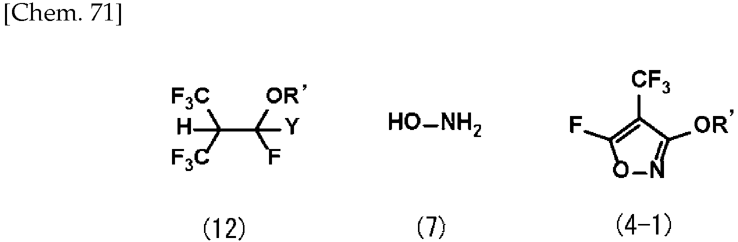 (In the above General Formulae (4-1) and (12),
   R' represents a substituted or unsubstituted hydrocarbon group having 1 to 12 carbon atoms,
   Y represents a halogen atom, -OB¹, -SOₘB¹, or -NB¹B²,
   m is an integer of 0 to 3, and
   B¹ and B² each independently represent a substituted or unsubstituted hydrocarbon group having 1 to 12 carbon atoms.)

Although the embodiments of the present invention have been described above, the present invention is not limited to the above embodiments, and includes all aspects included in the concept and claims of the present invention, and can be variously modified within the scope of the present invention.

### [Examples]

Hereinafter, examples of the present invention will be described, but the present invention is not limited to these examples as long as the present invention does not depart from the gist of the present invention. Furthermore, the room temperature described below is in the range of 20°C ± 10°C.

### (Example 1)

### Production of 3-Methoxy-4-(Trifluoromethyl)-5-Hydroxyisoxazole

Under ice-water cooling, 1.0 g (14 mmol) of hydroxylamine hydrochloride was dissolved in 75 g of methanol. Subsequently, 5.7 g (56 mmol) of triethylamine was added dropwise such that the internal temperature did not exceed 10°C. Next, 2.7 g (14 mmol) of methyl 3,3-difluoro-2-(trifluoromethyl)acrylate was added dropwise such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.2 g of a compound represented by the following Formula (16). The isolation yield of the compound obtained was 8%.

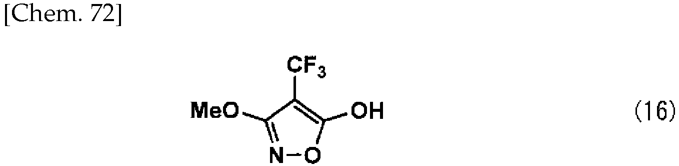

The analysis results are as follows.
Mass spectrum (APCI, m/z): 183 ([M]⁺)

### (Example 2)

### Production of 3-Ethoxy-4-(Trifluoromethyl)-5-Hydroxyisoxazole

Under ice-water cooling, 1.0 g (14 mmol) of hydroxylamine hydrochloride was dissolved in 75 g of ethanol. Subsequently, 5.7 g (56 mmol) of triethylamine was added dropwise such that the internal temperature did not exceed 10°C. Next, 2.7 g (14 mmol) of methyl 3,3-difluoro-2-(trifluoromethyl)acrylate was added dropwise such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.4 g of a compound represented by the following Formula (17). The isolation yield of the compound obtained was 13%.

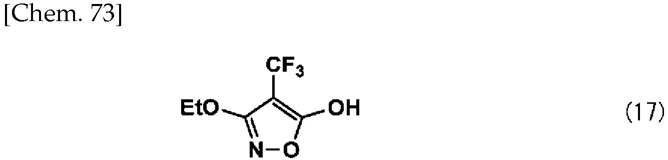

The analysis results are as follows.
Mass spectrum (APCI, m/z): 197 ([M]⁺)

### (Example 3)

### Production of 3-(Dibenzylamino)-4-(Trifluoromethyl)-5-Hydroxyisoxazole

Under ice-water cooling, 3.9 g (47 mmol) of 1-methylimidazole was added to 20 g of tetrahydrofuran. Subsequently, 10 g (47 mmol) of 1,3,3,3-tetrafluoro-1-methoxy-2-trifluoromethyl-1-propene was added dropwise such that the internal temperature did not exceed 10°C, and the temperature was raised to room temperature. After about 8 hours, a reaction mixture containing a salt of 3,3,3-trifluoro-2-(trifluoromethyl)propanoate fluoride anion and a 1,3-dimethylimidazolium cation was obtained. This reaction mixture was added dropwise to a mixed solution of 5.6 g (47 mmol) of 2-(2-methoxyethoxy)ethanol and 20 g of tetrahydrofuran under ice-water cooling such that the internal temperature did not exceed 10°C, and the temperature was raised to room temperature. After about 8 hours, a reaction mixture containing 2-(2-methoxyethoxy)ethyl 3,3,3-trifluoro-2-(trifluoromethyl)propanoate was obtained. This reaction mixture was added dropwise to a mixed solution of 9.3 g (47 mmol) of dibenzylamine, 9.5 g (94 mmol) of triethylamine, and 20 g of tetrahydrofuran under ice-water cooling such that the internal temperature did not exceed 10°C, and the temperature was raised to room temperature. After about 16 hours, a reaction mixture containing (E/Z)-3-(dibenzylamino)-3-fluoro-2-(trifluoromethyl)acrylic acid 2-(2-methoxyethoxy)ethyl ester was obtained. This reaction mixture was added dropwise to a mixed solution of 1.6 g (47 mmol) of hydroxylamine, 9.5 g (94 mmol) of triethylamine, and 20 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.5 g of a compound represented by the following Formula (18). The isolation yield of the compound obtained was 3%.

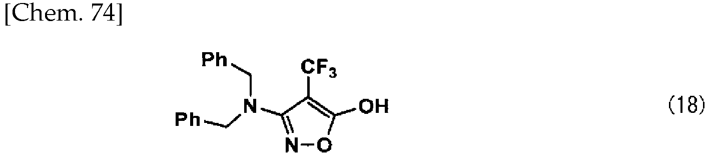

The analysis results are as follows.

### Mass spectrum (APCI, m/z): 348 ([M]⁺)

### (Example 4)

### Production of 3-(dibenzylamino)-4-(trifluoromethyl)-5-hydroxyisoxazole using methyl 3,3,3-trifluoro-2-(trifluoromethyl)propanoate Instead of 1,3,3,3-tetrafluoro-1-methoxy-2-trifluoromethyl-1-propene of Example 3

Under ice-water cooling, 1.9 g (9.5 mmol) of dibenzylamine and 1.9 g (19 mmol) of triethylamine were added to 30 g of tetrahydrofuran. Subsequently, 2.0 g (9.5 mmol) of methyl 3,3,3 trifluoro-2-(trifluoromethyl)propanoate was added dropwise such that the internal temperature did not exceed 10°C. After about 16 hours, a reaction mixture containing (E/Z)-3-(dibenzylamino)-3-fluoro-2-(trifluoromethyl)acrylic acid methyl ester was obtained. This reaction mixture was added dropwise to a mixed solution of 0.3 g (9.5 mmol) of hydroxylamine, 1.9 g (19 mmol) of triethylamine, and 30 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification. The analysis results were similar to those of the product of Example 3.

### (Example 5)

### Production of 3-(1-Azonanyl)-4-(Trifluoromethyl)-5-Hydroxyisoxazole

Under ice-water cooling, 3.6 g (30 mmol) of 2-(2-methoxyethoxy)ethanol was added to 30 g of tetrahydrofuran. Subsequently, 10 g (30 mmol) of a salt of 3,3,3-trifluoro-2-(trifluoromethyl)propanoate fluoride anion and 1-methyl-4-dimethylaminopyridinium cation was added dropwise to a mixed solution in which 30 g of tetrahydrofuran was dissolved such that the internal temperature did not exceed 10°C, and the temperature was raised to room temperature. After about 8 hours, a reaction mixture containing 2-(2-methoxyethoxy)ethyl 3,3,3-trifluoro-2-(trifluoromethyl)propanoate was obtained. This reaction mixture was added dropwise to a mixed solution of 3.8 g (30 mmol) of azonane, 6.1 g (60 mmol) of triethylamine, and 30 g of tetrahydrofuran under ice-water cooling such that the internal temperature did not exceed 10°C, and the temperature was raised to room temperature. After about 16 hours, a reaction mixture containing (E/Z)-3-(1-azonanyl)-3-fluoro-2-(trifluoromethyl)acrylic acid 2-(2-methoxyethoxy)ethyl ester was obtained. This reaction mixture was added dropwise to a mixed solution of 1.0 g (30 mmol) of hydroxylamine, 6.1 g (60 mmol) of triethylamine, and 30 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.9 g of a compound represented by the following Formula (19). The isolation yield of the compound obtained was 11%.

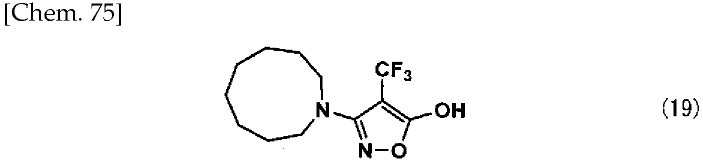

The analysis results are as follows.
Mass spectrum (APCI, m/z): 278 ([M]⁺)

### (Example 6)

### Production of 3-(1-Azonanyl)-4-(Trifluoromethyl)-5-Hydroxyisoxazole Using (E/Z)-3-(1-Azonanyl)-3-Fluoro-2-(Trifluoromethyl)Acrylic Acid Methyl Ester Instead of Salt of 3,3,3-Trifluoro-2-(Trifluoromethyl)Propanoate Fluoride Anion and 1-Methyl-4-Dimethylaminopyridinium Cation Of Example 5

Under ice-water cooling, 1.9 g (27 mmol) of hydroxylamine hydrochloride was dissolved in 60 g of methanol. Subsequently, 8.2 g (81 mmol) of triethylamine was added dropwise such that the internal temperature did not exceed 10°C. Next, 5 g (27 mmol) of (E/Z)-3-(1-azonanyl)-3-fluoro-2-(trifluoromethyl)acrylic acid methyl ester was added dropwise such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification. The analysis results were similar to those of the product of Example 5.

### (Example 7)

### Production of 3-(4,5,6,7-Tetrahydrothieno[3,2-c]Pyridin-5-Yl)-4-(Trifluoromethyl)-5-Hydroxyisoxazole

Under ice-water cooling, 20 g (86 mmol) of 1,1,1,3,3-pentafluoro-3-methoxy-2-trifluoromethyl-propane was added to 80 g of tetrahydrofuran. Subsequently, 11 g (86 mmol) of diisopropylethylamine was added dropwise such that the internal temperature did not exceed 10°C, and the temperature was raised to room temperature. After about 8 hours, a reaction mixture containing 1,3,3,3-tetrafluoro-1-methoxy-2-trifluoromethyl-1-propene was obtained. This reaction mixture was added dropwise to a mixed solution of 8.7 g (86 mmol) of triethylamine and 20 g of tetrahydrofuran under ice-water cooling such that the internal temperature did not exceed 10°C, and the temperature was raised to room temperature. After about 8 hours, a reaction mixture containing a salt of 3,3,3-trifluoro-2-(trifluoromethyl)propanoate fluoride anion and a triethylmethylammonium cation was obtained. This reaction mixture was added dropwise to a mixed solution of 10 g (86 mmol) of 2-(2-methoxyethoxy)ethanol and 20 g of tetrahydrofuran under ice-water cooling such that the internal temperature did not exceed 10°C, and the temperature was raised to room temperature. After about 8 hours, a reaction mixture containing 2-(2-methoxyethoxy)ethyl 3,3,3-trifluoro-2-(trifluoromethyl)propanoate was obtained. This reaction mixture was added dropwise to a mixed solution of 12 g (86 mmol) of 4,5,6,7-tetrahydrothieno[3,2-c]pyridine, 22 g (172 mmol) of diisopropylethylamine, and 40 g of tetrahydrofuran under ice-water cooling such that the internal temperature did not exceed 10°C, and the temperature was raised to room temperature. After about 16 hours, a reaction mixture containing (E/Z)-3-(4,5,6,7-tetrahydrothieno[3,2-c]pyridin-5-yl)-3-fluoro-2-(trifluoromethyl)acrylic acid 2-(2-methoxyethoxy)ethyl ester was obtained. This reaction mixture was added dropwise to a mixed solution of 2.8 g (86 mmol) of hydroxylamine, 22 g (172 mmol) of diisopropylethylamine, and 30 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.5 g of a compound represented by the following Formula (20). The isolation yield of the compound obtained was 2%.

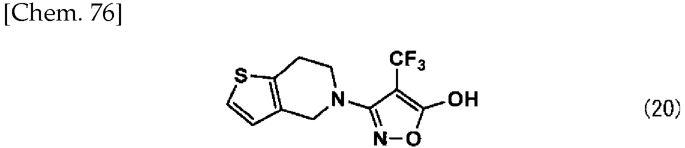

The analysis results are as follows.
Mass spectrum (APCI, m/z): 290 ([M]⁺)

### (Example 8)

Production of 3-Amino-N,N-Dibenzyl-5-Methoxy-4-(Trifluoromethyl)Isoxazole
under ice-water cooling, 1.1 g (3.0 mmol) of 3-(dibenzylamino)-3-fluoro-2-(trifluoromethyl)-2-propenoic acid methyl ester was added to 30 g of tetrahydrofuran, and 0.7 g (3.0 mmol) of 1-aminopyridinium iodide was further dissolved. Subsequently, 0.8 g (6.0 mmol) of diisopropylethylamine was added dropwise such that the internal temperature did not exceed 10°C, and the temperature was raised to room temperature. After about 16 hours, the contents were dissolved in a mixed solvent of hexane and ethyl acetate (hexane:ethyl acetate = 7:3), and subjected to silica gel column purification to obtain 0.2 g of a compound represented by the following Formula (21). The isolation yield of the compound obtained was 15%.

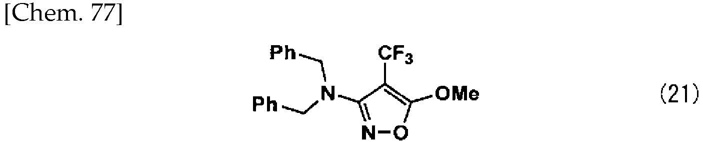

The analysis results are as follows.
Mass spectrum (APCI, m/z): 362 ([M]⁺)
¹H-NMR(400MHz, CDCl₃) δppm:7.33-7.24(m,10H), 4.65(s,4H), 3.86(s,3H)

### (Example 9)

### Production of 3-Fluoro-5-Methoxy-4-(Trifluoromethyl)Isoxazole

Under ice-water cooling, 0.6 g (3.0 mmol) of methyl 3,3 difluoro-2-(trifluoromethyl) acrylate was added to 20 g of tetrahydrofuran. Subsequently, a solution obtained by dissolving 0.9 g (3.0 mmol) of tetrabutylammonium azide in 20 g of tetrahydrofuran was added dropwise such that the internal temperature did not exceed 10°C, and the temperature was raised to room temperature. After about 72 hours, the contents were dissolved in a mixed solvent of hexane and ethyl acetate (hexane:ethyl acetate = 7:3), and subjected to silica gel column purification to obtain 0.1 g of a compound represented by the following Formula (22). The isolation yield of the compound obtained was 25%.

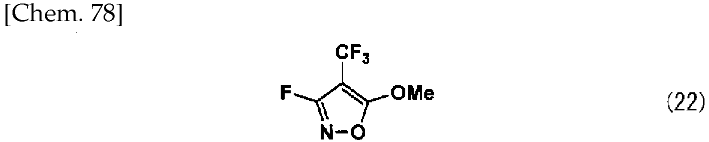

The analysis results are as follows.
Mass spectrum (APCI, m/z): 185 ([M]⁺)

### (Example 10)

### Production of 4-(3-(5-Methoxy-4-(Trifluoromethyl))Isoxazolyl)Thiomorpholine 1,1-Dioxide

Under ice-water cooling, 0.3 g (2.0 mmol) of thiomorpholine 1,1-dioxide and 0.5 g (4.0 mmol) of diisopropylethylamine were added to 20 g of tetrahydrofuran. Subsequently, 0.4 g (2.0 mmol) of methyl 3,3,3-trifluoro-2-(trifluoromethyl)propanoate was added dropwise such that the internal temperature did not exceed 10°C, and the temperature was raised to room temperature. After about 16 hours, the contents were cooled with ice-water, and 0.2 g (2.0 mmol) of hydroxylamine-O-sulfonic acid was added while ensuring that the internal temperature did not exceed 10°C, and subsequently, 0.8 g (6.0 mmol) of diisopropylethylamine was added dropwise, and the temperature was raised to room temperature. After about 36 hours, the contents were dissolved in a mixed solvent of hexane and ethyl acetate (hexane:ethyl acetate = 7:3), and subjected to silica gel column purification to obtain 0.1 g of a compound represented by the following Formula (23). The isolation yield of the compound obtained was 9%.

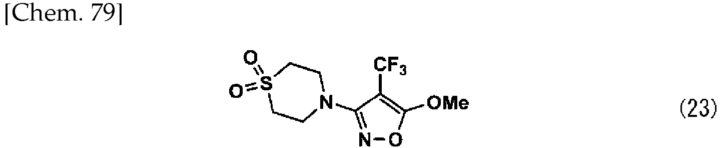

The analysis results are as follows.
Mass spectrum (APCI, m/z): 300 ([M]⁺)

### (Example 11)

### Production of 5-Ethoxy-3-((4-Methoxybenzyl)Oxy)-4-(Trifluoromethyl)Isoxazole

Under ice-water cooling, 0.5 g (10.0 mmol) of ethanol was added to 20 g of tetrahydrofuran. Subsequently, 3.0 g (9.0 mmol) of a salt of 3,3,3-trifluoro-2-(trifluoromethyl)propanoate fluoride anion and 1-methyl-4-dimethylaminopyridinium cation was added dropwise to a mixed solution in which 30 g of tetrahydrofuran was dissolved such that the internal temperature did not exceed 10°C, and the temperature was raised to room temperature. After about 8 hours, the reaction mixture was cooled with ice-water, and added dropwise to a mixed solution of 1.2 g (9.0 mmol) of 4-methoxybenzyl alcohol, 1.8 g (18.0 mmol) of triethylamine, and 30 g of tetrahydrofuran such that the internal temperature did not exceed 10°C, and the temperature was raised to room temperature. After about 16 hours, the reaction mixture was cooled with ice-water, 2.6 g (9.0 mmol) of 1-aminopyridinium mesitylenesulfonate was added such that the internal temperature did not exceed 10°C, subsequently 2.3 g (18.0 mmol) of diisopropylethylamine was added dropwise, and the temperature was raised to room temperature. After about 16 hours, the contents were dissolved in a mixed solvent of hexane and ethyl acetate (hexane:ethyl acetate = 7:3), and subjected to silica gel column purification to obtain 0.2 g of a compound represented by the following Formula (24). The isolation yield of the compound obtained was 8%.

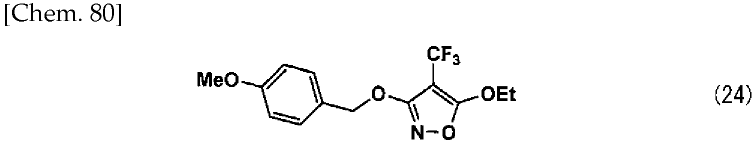

The analysis results are as follows.
Mass spectrum (APCI, m/z): 317 ([M]⁺)

### (Example 12)

### Production of 3-Amino-5-((2-Ethylhexyl)Oxy)-N-Methyl-N-(3-Pyridyl)-4-(Trifluoromethyl)Isoxazole

Under ice-water cooling, 3.9 g (47.0 mmol) of 1-methylimidazole was added to 20 g of tetrahydrofuran. Subsequently, 10 g (47.0 mmol) of 1,3,3,3-tetrafluoro-1-methoxy-2-trifluoromethyl-1-propene was added dropwise such that the internal temperature did not exceed 10°C, and the temperature was raised to room temperature. After about 8 hours, a reaction mixture containing a salt of 3,3,3-trifluoro-2-(trifluoromethyl)propanoate fluoride anion and a 1,3-dimethylimidazolium cation was obtained. This reaction mixture was added dropwise to a mixed solution of 6.1 g (47.0 mmol) of 2-ethyl-1-hexanol and 20 g of tetrahydrofuran under ice-water cooling such that the internal temperature did not exceed 10°C, and the temperature was raised to room temperature. After about 8 hours, a reaction mixture containing 2-ethylhexyl 3,3,3-trifluoro-2-(trifluoromethyl)propanoate was obtained. This reaction mixture was added dropwise to a mixed solution of 5.1 g (47.0 mmol) of 3-(methylamino)pyridine, 9.5 g (94.0 mmol) of triethylamine, and 20 g of tetrahydrofuran under ice-water cooling such that the internal temperature did not exceed 10°C, and the temperature was raised to room temperature. After about 16 hours, this reaction mixture was added dropwise to a mixed solution of 10 g (47.0 mmol) of o-(mesitylenesulfonyl)hydroxylamine, 9.5 g (94.0 mmol) of triethylamine, and 20 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.5 g of a compound represented by the following Formula (25). The isolation yield of the compound obtained was 3%.

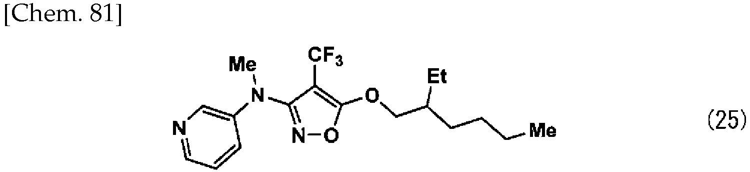

The analysis results are as follows.

### Mass spectrum (APCI, m/z): 371 ([M]⁺)

### (Example 13)

### Production of 4-(3-(5-(2-Methoxy) Thoxy-4-(Trifluoromethyl)Isoxazolyl)-2-Piperazinone

Under ice-water cooling, 20 g (86.0 mmol) of 1,1,1,3,3-pentafluoro-3-methoxy-2-trifluoromethyl-propane was added to 80 g of tetrahydrofuran. Subsequently, 11 g (86.0 mmol) of diisopropylethylamine was added dropwise such that the internal temperature did not exceed 10°C, and the temperature was raised to room temperature. After about 8 hours, a reaction mixture containing 1,3,3,3-tetrafluoro-1-methoxy-2-trifluoromethyl-1-propene was obtained. This reaction mixture was added dropwise to a mixed solution of 9.6 g (86.0 mmol) of quinuclidine and 20 g of tetrahydrofuran under ice-water cooling such that the internal temperature did not exceed 10°C, and the temperature was raised to room temperature. After about 8 hours, a reaction mixture containing a salt of 3,3,3-trifluoro-2-(trifluoromethyl)propanoate fluoride anion and a N-methylquinuclidinium cation was obtained. This reaction mixture was added dropwise to a mixed solution of 6.5 g (86.0 mmol) of 2-methoxyethanol and 20 g of tetrahydrofuran under ice-water cooling such that the internal temperature did not exceed 10°C, and the temperature was raised to room temperature. After about 8 hours, a reaction mixture containing 2-methoxyethyl 3,3,3-trifluoro-2-(trifluoromethyl)propanoate was obtained. This reaction mixture was added dropwise to a mixed solution of 8.6 g (86.0 mmol) of piperazinone, 22 g (172.0 mmol) of diisopropylethylamine, and 40 g of tetrahydrofuran under ice-water cooling such that the internal temperature did not exceed 10°C, and the temperature was raised to room temperature. After about 16 hours, this reaction mixture was added dropwise to a mixed solution of 16 g (86.0 mmol) of 1,1,1-trimethylhydrazinium trifluoroacetate, 22 g (172.0 mmol) of diisopropylethylamine, and 30 g of tetrahydrofuran under ice-water cooling such that the internal temperature did not exceed 10°C, and the temperature was raised to room temperature. After about 16 hours, a 1 N aqueous hydrochloric acid solution was added such that the pH of the reaction mixture was about 5, and ammonium chloride was added until saturated. The organic phase was dried over sodium sulfate, then dissolved in a mixed solvent of hexane and ethyl acetate (hexane:ethyl acetate = 7:3), and subjected to silica gel column purification to obtain 0.5 g of a compound represented by the following Formula (26). The isolation yield of the compound obtained was 2%.

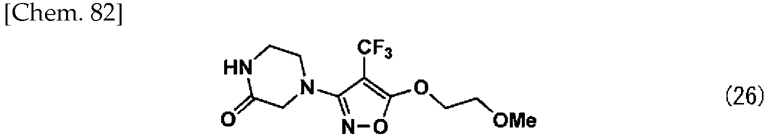

The analysis results are as follows.
Mass spectrum (APCI, m/z): 309 ([M]⁺)

### (Example 14)

### Production of 5-(1H-Benzo[d][1,2,3]Triazole-1-Yl)-4-(Trifluoromethyl)Isoxazole-3-Ol

Under ice-water cooling, 2 g (6.4 mmol) of hydroxylammonium bis(trifluoromethanesulfonyl)imide was dissolved in 30 g of tetrahydrofuran. Subsequently, a mixed solution in which 1.9 g (6.4 mmol) of a salt of 3,3,3-trifluoro-2-(trifluoromethyl)propanoate fluorido anion and an N-methylimidazolium cation was dissolved in 20 g of tetrahydrofuran was added dropwise such that the internal temperature did not exceed 10°C, and the temperature was raised to room temperature. After about 3 hours, the reaction mixture was cooled with ice-water, 0.7 g (6.4 mmol) of triethylamine was added dropwise such that the internal temperature did not exceed 10°C, and the temperature was raised to room temperature. After about 4 hours, the reaction mixture was cooled with ice-water, and added dropwise to a mixed solution of 0.8 g (6.4 mmol) of 1,2,3-benzotriazole, 2.3 g (23.0 mmol) of triethylamine, and 30 g of tetrahydrofuran such that the internal temperature did not exceed 10°C, and the temperature was raised to room temperature. After about 16 hours, the contents were dissolved in a mixed solvent of hexane and ethyl acetate (hexane:ethyl acetate = 7:3), and subjected to silica gel column purification to obtain 0.3 g of a compound represented by the following Formula (27). The isolation yield of the compound obtained was 17%.

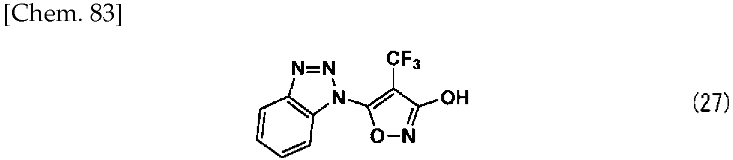

The analysis results are as follows.
Mass spectrum (APCI, m/z): 270 ([M]⁺)
¹H-NMR (400MHz, CDCl₃) δppm:8.19 (dd, 1H), 8.12 (dd, 1H), 7.41 (ddd, 1H), 7.33(ddd, 1H)

### (Example 15)

### Production of 5-(10H-Spiro[Acridine-9,9'-Fluorene]-10-Yl)-4-(Trifluoromethyl)Isoxazole-3-Ol

Under ice-water cooling, 0.9 g (8.5 mmol) of triethylamine was added to 20 g of acetonitrile. Subsequently, 1.8 g (8.5 mmol) of 1,3,3,3-tetrafluoro-1-methoxy-2-trifluoromethyl-1-propene was added dropwise such that the internal temperature did not exceed 10°C, and the temperature was raised to room temperature. After about 4 hours, the reaction mixture was added dropwise to a mixed solution of 3.0 g (8.5 mmol) of hydroxylammonium tetraphenylborate and 20 g of acetonitrile under ice-water cooling such that the internal temperature did not exceed 10°C, and the temperature was raised to room temperature. After about 3 hours, the reaction mixture was cooled with ice-water, 0.9 g (8.5 mmol) of triethylamine was added dropwise such that the internal temperature did not exceed 10°C, and the temperature was raised to room temperature. After about 4 hours, the reaction mixture was added dropwise to a mixed solution of 2.8 g (8.5 mmol) of 10H-spiro[acridine-9, 9'-fluorene], 3.0 g (30.0 mmol) of triethylamine, and 20 g of acetonitrile under ice-water cooling such that the internal temperature did not exceed 10°C, and the temperature was raised to room temperature. After about 16 hours, the contents were dissolved in a mixed solvent of hexane and ethyl acetate (hexane:ethyl acetate = 7:3), and subjected to silica gel column purification to obtain 0.4 g of a compound represented by the following Formula (27). The isolation yield of the compound obtained was 10%.

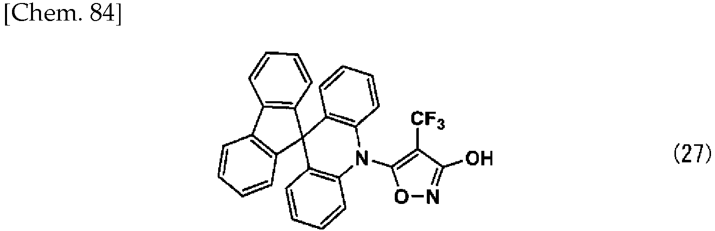

The analysis results are as follows.
Mass spectrum (APCI, m/z): 482 ([M]⁺)

### (Example 16)

### Production of 5-((2,2,4,4-Tetramethylpentane-3-Ylidene) Amino)-4-(Trifluoromethyl)Isoxazole-3-Ol

Under ice-water cooling, 1.1 g (4.8 mmol) of 1,1,1,3,3-pentafluoro-3-methoxy-2-trifluoromethyl-propane was added to 40 g of tetrahydrofuran. Subsequently, 0.6 g (4.8 mmol) of diisopropylethylamine was added dropwise such that the internal temperature did not exceed 10°C, and the temperature was raised to room temperature. After about 4 hours, the reaction mixture was added dropwise to a mixed solution of 0.6 g (4.8 mmol) of 4-dimethylaminopyridine and 15 g of tetrahydrofuran under ice-water cooling such that the internal temperature did not exceed 10°C, and the temperature was raised to room temperature. After about 4 hours, the reaction mixture was added dropwise to a mixed solution of 1.5 g (4.8 mmol) of hydroxylammonium bis(trifluoromethanesulfonyl)imide and 20 g of tetrahydrofuran under ice-water cooling such that the internal temperature did not exceed 10°C, and the temperature was raised to room temperature. After about 3 hours, the reaction mixture was cooled with ice-water, 0.6 g (4.8 mmol) of diisopropylethylamine was added dropwise such that the internal temperature did not exceed 10°C, and the temperature was raised to room temperature. After about 4 hours, the reaction mixture was added dropwise to a mixed solution of 0.7 g (4.8 mmol) of 2,2,4,4-tetramethyl-3-pentanone imine, 2.8 g (22.0 mmol) of diisopropylethylamine, and 20 g of tetrahydrofuran under ice-water cooling such that the internal temperature did not exceed 10°C, and the temperature was raised to room temperature. After about 16 hours, the contents were dissolved in a mixed solvent of hexane and ethyl acetate (hexane:ethyl acetate = 7:3), and subjected to silica gel column purification to obtain 0.1 g of a compound represented by the following Formula (28). The isolation yield of the compound obtained was 9%.

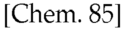

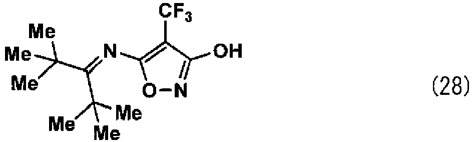

The analysis results are as follows.
Mass spectrum (APCI, m/z): 292 ([M]⁺)

### (Example 17)

### Production of 5-Fluoro-3-Methoxy-4-Trifluoromethyl Isoxazole

Under ice-water cooling, 3.1 g (10.0 mmol) of hydroxylammonium bis(trifluoromethylsulfonyl)amine and 2.1 g (10.0 mmol) of 1,3,3,3-tetrafluoro-1-methoxy-2-trifluoromethyl-1-propene were added to 75 g of 4-methyltetrahydropyran. Subsequently, a solution obtained by dissolving 4.7 g (20.0 mmol) of tert-butylimino-tris(dimethylamino)phosphorane in 25 g of 4-methyltetrahydropyran was further added dropwise such that the internal temperature did not exceed 10°C, and the temperature was raised to room temperature. After about 16 hours, the mixture was cooled with ice-water, a solution obtained by dissolving 6.1 g (26.0 mmol) of tert-butylimino-tris(dimethylamino)phosphorane in 20 g of 4-methyltetrahydropyran was further added dropwise such that the internal temperature did not exceed 10°C, and the temperature was raised to room temperature. After about 8 hours, the reaction mixture was dissolved in a mixed solvent of hexane and ethyl acetate (hexane: ethyl acetate =7:3), and subjected to silica gel column purification to obtain 0.04 g of a compound represented by the following Formula (29). The isolation yield of the compound obtained was 2%.

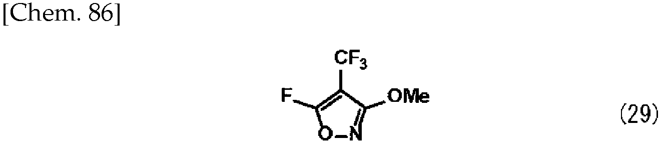

The analysis results are as follows.
Mass spectrum (APCI, m/z): 185 ([M]⁺)

### (Example 18)

### Production of 3-Methoxy-5-Morpholinyl-4-Trifluoromethylisoxazole

Under ice-water cooling, 0.7 g (10.0 mmol) of hydroxylamine hydrochloride and 2.1 g (10.0 mmol) of 1,3,3,3-tetrafluoro-1-methoxy-2-trifluoromethyl-1-propene were added to 30 g of methanol. Subsequently, a solution obtained by dissolving 2.0 g (20.0 mmol) of triethylamine in 10 g of methanol was added dropwise such that the internal temperature did not exceed 10°C, and the temperature was raised to room temperature. After about 16 hours, the mixture was cooled with ice-water, a solution obtained by dissolving 4.5 g (52.0 mmol) of morpholine in 15 g of methanol was further added dropwise such that the internal temperature did not exceed 10°C, and the temperature was raised to room temperature. After about 8 hours, the reaction mixture was dissolved in a mixed solvent of hexane and ethyl acetate (hexane: ethyl acetate =7:3), and subjected to silica gel column purification to obtain 0.5 g of a compound represented by the following Formula (30). The isolation yield of the compound obtained was 18%.

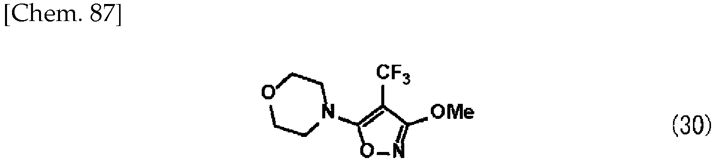

The analysis results are as follows.
Mass spectrum (APCI, m/z): 252 ([M]⁺)
¹H-NMR (400MHz, CDCl₃) δppm:3.80 (m,7H), 3.50 (dd,4H)

### (Example 19)

### Production of 5-Fluoro-3-Methoxy-4-Trifluoromethylisoxazole Using 1,1,1,3,3-Pentafluoro-3-Methoxy-2-(Trifluoromethyl)Propane Instead of 1,3,3,3-Tetrafluoro-1-Methoxy-2-(Trifluoromethyl)-1-Propene of Example 17

Under ice-water cooling, 3.1 g (10 mmol) of hydroxylammonium bis(trifluoromethylsulfonyl)amine and 23 g (10 mmol) of 1,1,1,3,3-Pentafluoro-3-methoxy-2-(trifluoromethyl)propane were added to 75 g of 4-methyltetrahydropyran. Subsequently, a solution obtained by dissolving 7.1 g (30 mmol) of tert-butylimino-tris(dimethylamino)phosphorane in 25 g of 4-methyltetrahydropyran was further added dropwise such that the internal temperature did not exceed 10°C, and the temperature was raised to room temperature. After about 16 hours, the mixture was cooled with ice-water, a solution obtained by dissolving 6.1 g (26 mmol) of tert-butylimino-tris(dimethylamino)phosphorane in 20 g of 4-methyltetrahydropyran was further added dropwise such that the internal temperature did not exceed 10°C, and the temperature was raised to room temperature. After about 8 hours, the reaction mixture was dissolved in a mixed solvent of hexane and ethyl acetate (hexane: ethyl acetate =7:3), and subjected to silica gel column purification. Analysis results of the obtained compound were similar to those of the product of Example 17.

Furthermore, in Example 19, the isolation yield of the obtained compound is not calculated, but due to a by-product that can be generated in the process of producing 1,3,3,3-tetrafluoro-1-methoxy-2-(trifluoromethyl)-1-propene from 1,3,3,3-tetrafluoro-1-methoxy-2-(trifluoromethyl)-1-propene in the system, the type and amount of impurities are predicted to increase. Therefore, it is considered that the isolation yield of the obtained product is higher in the production method of Example 17 than in the corresponding production method of Example 19.

### (Example 20)

### Production of 5-(1-Benzimidazolyl)-3-Methoxy-4-Trifluoromethylisoxazole

Under ice-water cooling, 1.0 g (14 mmol) of hydroxylamine hydrochloride and 3.1 g (14 mmol) of 1,3,3,3-tetrafluoro-1-methoxy-2-trifluoromethyl-1-propene were added to 45 g of methanol. Subsequently, a solution obtained by dissolving 2.9 g (28 mmol) of triethylamine in 20 g of methanol was added dropwise such that the internal temperature did not exceed 10°C, and the temperature was raised to room temperature. After about 16 hours, the mixture was cooled with ice- water, a solution obtained by dissolving 1.7 g (14 mmol) of benzimidazole and 4.4 g (42 mmol) of triethylamine in 20 g of methanol was further added dropwise such that the internal temperature did not exceed 10°C, and the temperature was raised to room temperature. After about 8 hours, the reaction mixture was dissolved in a mixed solvent of hexane and ethyl acetate (hexane: ethyl acetate =7:3), and subjected to silica gel column purification to obtain 0.6 g of a compound represented by the following Formula (31). The isolation yield of the compound obtained was 15%.

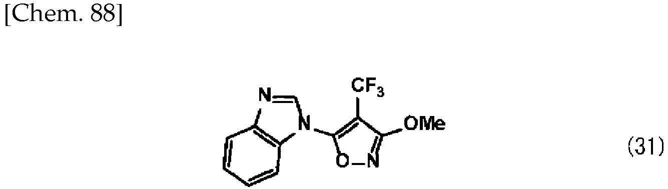

The analysis results are as follows.
Mass spectrum (APCI, m/z): 283 ([M]⁺)
¹H-NMR (400MHz, CDCl₃) δ ppm: 8.21 (s, 1H), 7.90 (d, 1H), 7.70 (d, 1H), 7.46 (dd, 2H), 4.16 (m, 3H)

### (Example 21)

### Production of 3-Methoxy-5-(N-Methoxymethylamino)-4-Trifluoromethylisoxazole

Under ice-water cooling, 1.0 g (14 mmol) of hydroxylamine hydrochloride and 3.1 g (14 mmol) of 1,3,3,3-tetrafluoro-1-methoxy-2-trifluoromethyl-1-propene were added to 45 g of methanol. Subsequently, a solution obtained by dissolving 2.9 g (28 mmol) of triethylamine in 20 g of methanol was added dropwise such that the internal temperature did not exceed 10°C, and the temperature was raised to room temperature. After about 16 hours, the mixture was cooled with ice- water, a solution obtained by dissolving 1.4 g (14 mmol) of N-methoxymethylamine hydrochloride and 5.8 g (56 mmol) of triethylamine in 20 g of methanol was further added dropwise such that the internal temperature did not exceed 10°C, and the temperature was raised to room temperature. After about 8 hours, the reaction mixture was dissolved in a mixed solvent of hexane and ethyl acetate (hexane: ethyl acetate =7:3), and subjected to silica gel column purification to obtain 0.3 g of a compound represented by the following Formula (32). The isolation yield of the compound obtained was 8%.

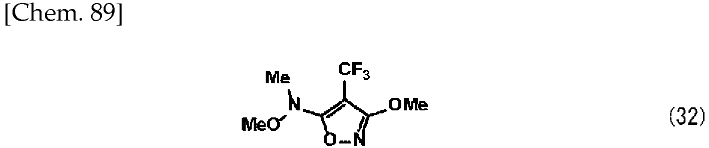

The analysis results are as follows.
Mass spectrum (APCI, m/z): 226 ([M]⁺)
¹H-NMR (400MHz, CDCl₃) δppm:3.98 (s, 3H), 3.74 (s, 3H), 3.21 (s, 3H)

### (Example 22)

### Production of 5-[(N-Diphenylmethylene)Amino]-3-Methoxy-4-Trifluoromethylisoxazole

0.5 g (7.3 mmol) of hydroxylamine hydrochloride and 1.5 g (7.1 mmol) of 1,3,3,3-tetrafluoro-1-methoxy-2-trifluoromethyl-1-propene were added to 14 ml of methanol. Subsequently, after cooling to 0°C, 1.5 g (14.6 mmol) of triethylamine was added dropwise, and the temperature was raised to room temperature. After about 19 hours, the mixture was cooled to 0°C, 1.3 g (7.2 mmol) of benzophenoneimine and 2.2 g (21.8 mmol) of triethylamine were added dropwise, and the temperature was raised to 60°C. After about 7 hours, the mixture was cooled and subjected to silica gel column purification to obtain a trace amount of a compound represented by the following Formula (33).

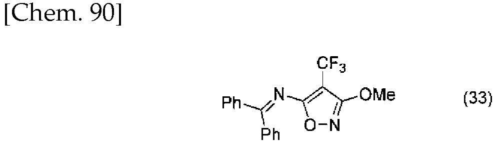

The analysis results are as follows.
Mass spectrum (APCI, m/z): 346 ([M]⁺)

## Claims

1. A fluorine-containing isoxazole compound represented by General Formula (A) below. 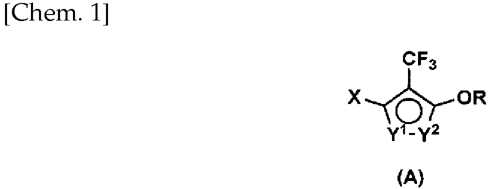 (In the above General Formula (A),
R represents a hydrogen atom or a substituted or unsubstituted hydrocarbon group having 1 to 12 carbon atoms,
X represents a halogen atom, -OA¹, -O-NA¹A², -NA¹A², -NA^{I}(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

2. The fluorine-containing isoxazole compound according to claim 1, wherein the fluorine-containing isoxazole compound is a compound represented by General Formula (1), (2), (3), or (4) below. 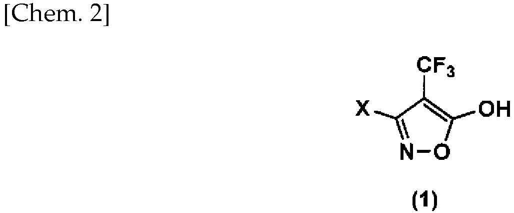 (In the above General Formula (1),
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.) 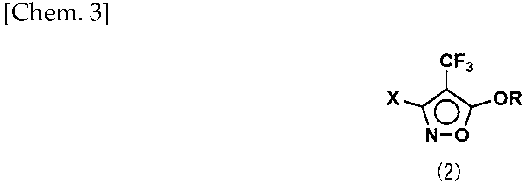
(In the above General Formula (2),
R represents a substituted or unsubstituted hydrocarbon having 1 to 12 carbon atoms, and X represents a halogen atom, -OA¹, -O-NA¹A², -NA¹A², - NA¹(OA²), -NA³-NA¹A², -NA⁴, or a heterocycle;
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.) 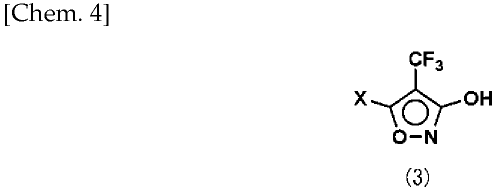
(In the above General Formula (3),
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.) 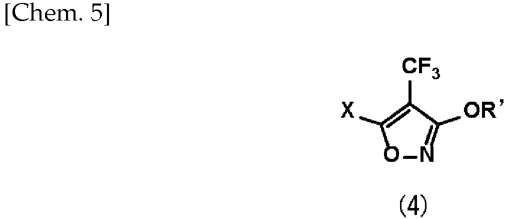
(In the above General Formula (4),
R' represents a substituted or unsubstituted hydrocarbon having 1 to 12 carbon atoms,
X represents a halogen atom, -OA¹, -O-NA¹A², -NA¹A², -NA^{I}(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ, and
n is an integer of 1 to 20.)

3. A method for producing a fluorine-containing isoxazole compound, the method comprising:
a step of synthesizing a fluorine-containing isoxazole compound represented by General Formula (A) below
(I) by reacting a fluorine-containing compound selected from a fluorine-containing carbonyl compound, a fluoroisobutene derivative, and a fluoroisobutane derivative, hydroxylamine or a salt thereof, and optionally a compound represented by General Formula (5) below or a salt thereof, or
(II) by reacting a fluorine-containing compound selected from a fluorine-containing carbonyl compound, a fluoroisobutene derivative, and a fluoroisobutane derivative, an amine salt or an amine compound, and optionally a compound represented by General Formula (5) below or a salt thereof. 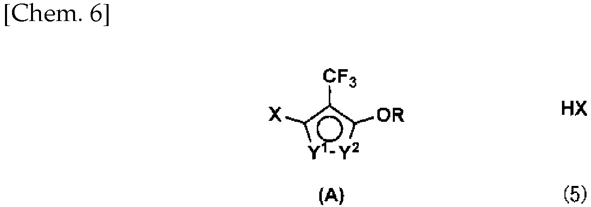 (In the above General Formulae (A) and (5),
R represents a hydrogen atom or a substituted or unsubstituted hydrocarbon group having 1 to 12 carbon atoms,
X represents a halogen atom, -OA¹, -O-NA¹A², -NA¹A², -NA^{I}(OA²), -NA³-NA¹A², -NA⁴, or a heterocycle,
A¹, A², and A³ each independently represent a substituted or unsubstituted hydrocarbon group having 1 to 12 carbon atoms or a heterocycle,
A⁴ represents an alkylene group represented by =CₙH₂ₙ,
n is an integer of 1 to 20, and
Y¹ and Y² each independently represent N or O, and are different from each other.)

4. The method for producing a fluorine-containing isoxazole compound according to claim 3, the method comprising:
a step of obtaining a fluorine-containing isoxazole compound represented by General Formula (1) below by reacting a fluorine-containing carbonyl compound represented by General Formula (6) below with hydroxylamine represented by General Formula (7) below or a salt thereof. 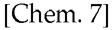 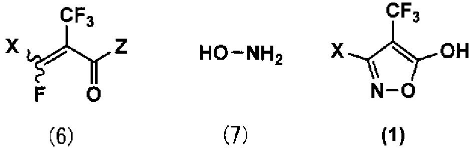
(In the above General Formulae (1), (6), and (7),
X represents a halogen atom, -OA¹, -O-NA¹A², -NA¹A², -NA^{I}(OA²), -NA³-NA¹A², -NA⁴, or a heterocycle,
Z represents -OA⁵ , -O-NA⁵A⁶, -NA⁵A⁶, -NA⁵(OA⁶), or -NA⁷-NA⁵A⁶,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20,
A⁵, A⁶, and A⁷ each independently represent a substituted or unsubstituted hydrocarbon group having 1 to 12 carbon atoms, and
a wavy bond indicates a single bond representing stereoisomerism of (E) or (Z).)

5. The method for producing a fluorine-containing isoxazole compound according to claim 3, the method comprising:
a step of obtaining a fluorine-containing isoxazole compound represented by General Formula (1) below by reacting a fluorine-containing carbonyl compound represented by General Formula (8) below, a compound represented by General Formula (5) below or a salt thereof, and a hydroxylamine represented by General Formula (7) below or a salt thereof. 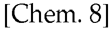 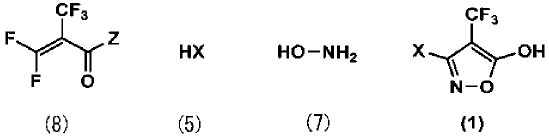
(In the above General Formulae (1), (5), and (8),
X represents a halogen atom, -OA¹, -O-NA¹A², -NA¹A², -NA^{I}(OA²), -NA³-NA¹A², -NA⁴, or a heterocycle,
Z represents -OA⁵ , -O-NA⁶A⁷, -NA⁵A⁶, -NA⁵(OA⁶), or -NA⁷-NA⁵A⁶, and A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
A⁵, A⁶, and A⁷ each independently represent a substituted or unsubstituted hydrocarbon group having 1 to 12 carbon atoms.)

6. The method for producing a fluorine-containing isoxazole compound according to claim 3, the method comprising:
a step of obtaining a fluorine-containing isoxazole compound represented by General Formula (1) below by reacting a fluorine-containing carbonyl compound represented by General Formula (9) below with a compound represented by General Formula (5) below or a salt thereof in the presence of a base, and then further reacting the obtained reaction product with hydroxylamine represented by General Formula (7) below or a salt thereof. 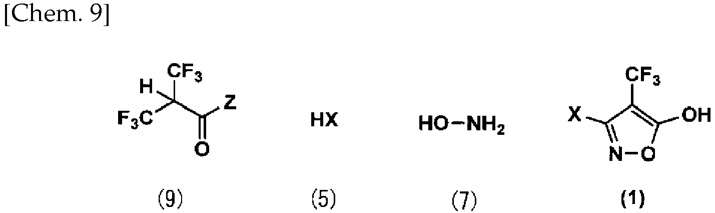
(In the above General Formulae (1), (5), and (9),
X represents a halogen atom, -OA¹, -O-NA¹A², -NA¹A², -NA¹(OA²), -NA³-NA¹A², -NA⁴, or a heterocycle,
Z represents -OA⁵, -O-NA⁶A⁷, -NA⁵A⁶, -NA⁵(OA⁶), or -NA⁷-NA⁵A⁶,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
A⁵, A⁶, and A⁷ each independently represent a substituted or unsubstituted hydrocarbon group having 1 to 12 carbon atoms.)

7. The method for producing a fluorine-containing isoxazole compound according to claim 3, the method comprising:
a step of obtaining a fluorine-containing isoxazole compound represented by General Formula (1) below by reacting a fluorine-containing carbonyl compound represented by General Formula (10) below with an alcohol, then reacting the obtained reaction product with a compound represented by General Formula (5) below or a salt thereof in the presence of a base, and then reacting the obtained reaction product with hydroxylamine represented by General Formula (7) below or a salt thereof. 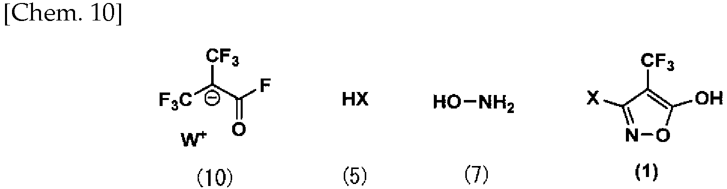
(In the above General Formulae (1), (5), and (10),
X represents a halogen atom, -OA¹, -O-NA¹A², -NA¹A², -NA^{I}(OA²), -NA³-NA¹A², -NA⁴, or a heterocycle,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

8. The method for producing a fluorine-containing isoxazole compound according to claim 3, the method comprising:
a step of obtaining a fluorine-containing isoxazole compound represented by General Formula (1) below by carbonylating a fluoroisobutene derivative represented by General Formula (11) below in the presence of a nucleophile, then reacting the fluoroisobutene derivative with an alcohol, and then reacting the obtained reaction product with a compound represented by General Formula (5) below or a salt thereof in the presence of a base, and then further reacting the obtained reaction product with hydroxylamine represented by General Formula (7) below or a salt thereof. 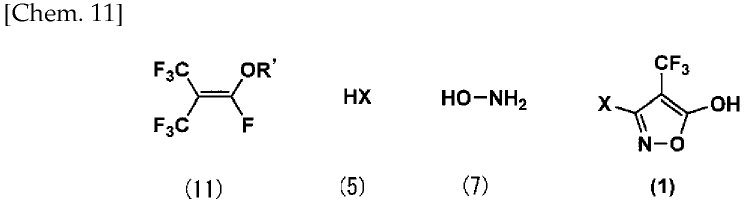
(In the above General Formulae (1), (5), and (11),
R' represents a substituted or unsubstituted hydrocarbon group having 1 to 12 carbon atoms,
X represents a halogen atom, -OA¹, -O-NA¹A², -NA^{I}(OA²), -NA¹A², -NA³-NA¹A², -NA⁴, or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

9. The method for producing a fluorine-containing isoxazole compound according to claim 3, the method comprising:
a step of obtaining a fluorine-containing isoxazole compound represented by General Formula (1) below by carbonylating a compound obtained by an elimination reaction of a fluoroisobutane derivative represented by General Formula (12) below in the presence of a nucleophile, then reacting the fluoroisobutane derivative with an alcohol, and then reacting the obtained reaction product with a compound represented by General Formula (5) below or a salt thereof in the presence of a base, and then further reacting the obtained reaction product with hydroxylamine represented by General Formula (7) below or a salt thereof. 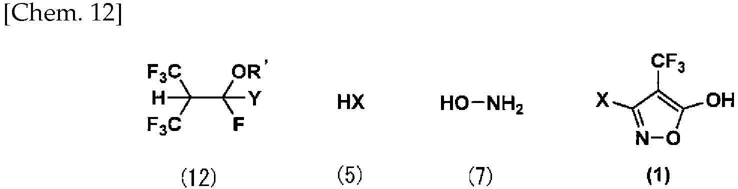
(In the above General Formulae (1), (5), and (12),
R' represents a substituted or unsubstituted hydrocarbon group having 1 to 12 carbon atoms,
X represents a halogen atom, -OA¹, -O-NA¹A², -NA¹A², -NA^{I}(OA²), -NA³-NA¹A², -NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

10. The method for producing a fluorine-containing isoxazole compound according to claim 3, the method comprising:
a step of obtaining a fluorine-containing isoxazole compound represented by General Formula (2) below by reacting a fluorine-containing carbonyl compound represented by General Formula (13) below with an amine salt or an amine compound. 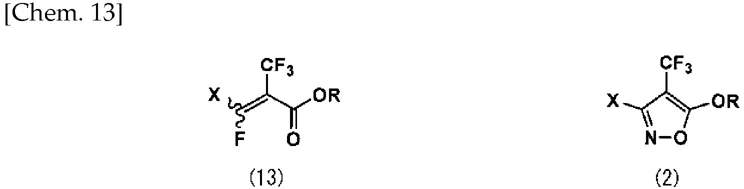
(In the above General Formulae (2) and (13),
R represents a substituted or unsubstituted hydrocarbon group having 1 to 12 carbon atoms,
X represents -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
a wavy bond indicates a single bond representing stereoisomerism of (E) or (Z).)

11. The method for producing a fluorine-containing isoxazole compound according to claim 3, the method comprising:
a step of obtaining a fluorine-containing isoxazole compound represented by General Formula (2) below by reacting a fluorine-containing carbonyl compound represented by General Formula (14) below with an amine salt or an amine compound, and optionally a compound represented by General Formula (5) below or a salt thereof. 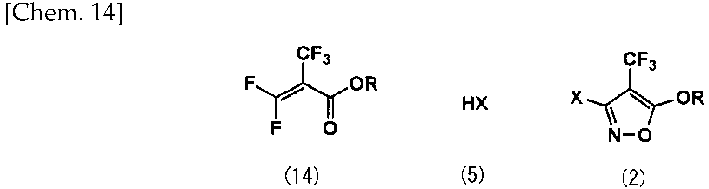
(In the above General Formulae (2), (5), and (14),
R represents a substituted or unsubstituted hydrocarbon group having 1 to 12 carbon atoms,
X represents a halogen atom, -OA¹, -O-NA¹A², -NA¹(OA²), -NA¹A², -NA³-NA¹A², -NA⁴, or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

12. The method for producing a fluorine-containing isoxazole compound according to claim 3, the method comprising:
a step of obtaining a fluorine-containing isoxazole compound represented by General Formula (2) below by reacting a fluorine-containing carbonyl compound represented by General Formula (15) below with a compound represented by General Formula (5) below and a salt thereof in the presence of a base, and then reacting the obtained reaction product with an amine salt or an amine compound. 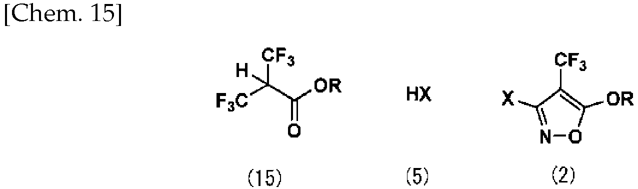
(In the above General Formulae (2), (5), and (15),
R represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a hydrogen atom, a substituted or unsubstituted hydrocarbon group having 1 to 12 carbon atoms, or a heterocyclic ring,
A⁴ represents an alkylene group represented by =CₙH₂ₙ, and
n is an integer of 1 to 20.)

13. The method for producing a fluorine-containing isoxazole compound according to claim 3, the method comprising:
a step of obtaining a fluorine-containing isoxazole compound represented by General Formula (2) below by reacting a fluorine-containing carbonyl compound represented by General Formula (10) below with an alcohol, then reacting the obtained reaction product with a compound represented by General Formula (5) below or a salt thereof in the presence of a base, and then reacting the obtained reaction product with an amine salt or an amine compound. 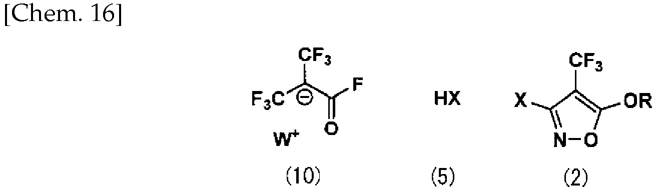
(In the above General Formulae (2), (5), and (10),
R represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

14. The method for producing a fluorine-containing isoxazole compound according to claim 3, the method comprising:
a step of obtaining a fluorine-containing isoxazole compound represented by General Formula (2) below by carbonylating a fluoroisobutene derivative represented by General Formula (11) below in the presence of a nucleophile, then reacting the fluoroisobutene derivative with an alcohol, and then reacting the obtained reaction product with a compound represented by General Formula (5) below or a salt thereof in the presence of a base, and then reacting the obtained reaction product with an amine salt or an amine compound. 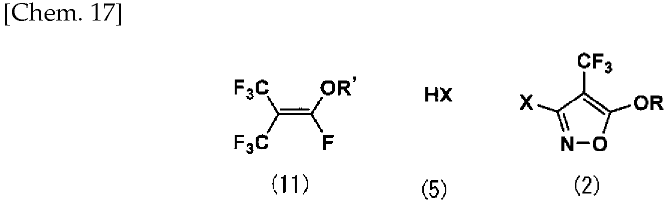
(In the above General Formulae (2), (5), and (11),
R and R' each independently represent a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

15. The method for producing a fluorine-containing isoxazole compound according to claim 3, the method comprising:
a step of obtaining a fluorine-containing isoxazole compound represented by General Formula (2) below by carbonylating a compound obtained by an elimination reaction of a fluoroisobutane derivative represented by General Formula (12) below in the presence of a nucleophile, then reacting the fluoroisobutane derivative with an alcohol, and then reacting the obtained reaction product with a compound represented by General Formula (5) below or a salt thereof in the presence of a base, and then reacting the obtained reaction product with an amine salt or an amine compound. 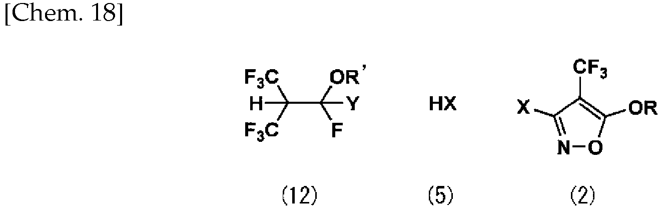
(In the above General Formulae (2), (5), and (12),
R and R' each independently represent a substituted or unsubstituted hydrocarbon group having 1 to 12 carbon atoms, and X represents a halogen atom, -OA¹, -O-NA¹A², -NA¹A², -NA¹(OA²), -NA³-NA¹A²-NA⁴, or a heterocycle,
Y represents a halogen atom, -OB¹, -SOₘB¹, or -NB¹B²,
m is an integer of 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

16. The method for producing a fluorine-containing isoxazole compound according to claim 3, the method comprising:
a step of obtaining a fluorine-containing isoxazole compound represented by General Formula (3) below by reacting a fluorine-containing carbonyl compound represented by General Formula (10) below with hydroxylamine represented by General Formula (7) below and a salt thereof, and further reacting the obtained reaction product with a compound represented by General Formula (5) or a salt thereof. 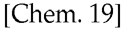 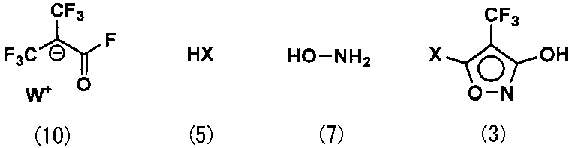
(In the above General Formulae (3), (5), and (10),
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms, or a heterocyclic ring,
A⁴ represents an alkylene group represented by =CₙH₂ₙ,
n is an integer of 1 to 20, and
W⁺ represents an ammonium cation, an imidazolium cation, a pyridinium cation, a quinuclidinium cation, or a phosphonium cation.)

17. The method for producing a fluorine-containing isoxazole compound according to claim 3, the method comprising:
a step of obtaining a fluorine-containing isoxazole compound represented by General Formula (3) below by carbonylating a fluoroisobutene derivative represented by General Formula (11) below in the presence of a nucleophile, then further reacting the fluoroisobutene derivative with hydroxylamine represented by General Formula (7) and a salt thereof, and then reacting the obtained reaction product with a compound represented by General Formula (5) below or a salt thereof. 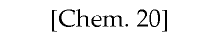 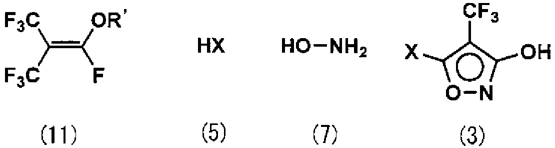
(In the above General Formulae (3), (5), and (11),
R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

18. The method for producing a fluorine-containing isoxazole compound according to claim 3, the method comprising:
a step of obtaining a fluorine-containing isoxazole compound represented by General Formula (3) below by carbonylating a fluoroisobutane derivative represented by General Formula (12) below in the presence of a nucleophile, further reacting the fluoroisobutane derivative with hydroxylamine represented by General Formula (7) below or a salt thereof, and then reacting the obtained reaction product with a compound represented by General Formula (5) below or a salt thereof. 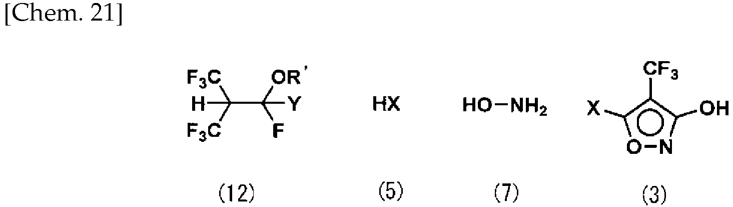
(In the above General Formulae (3), (5), and (12),
R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A²-NA⁴, or a heterocycle,
Y represents a halogen atom, -OB¹, -SOₘB¹, or -NB¹B²,
m is an integer from 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

19. The method for producing a fluorine-containing isoxazole compound according to claim 3, the method comprising:
a step of obtaining a fluorine-containing isoxazole compound represented by General Formula (4) below by reacting a fluoroisobutene derivative represented by General Formula (11) below, a compound represented by General Formula (5) below or a salt thereof, and hydroxylamine represented by General Formula (7) below or a salt thereof. 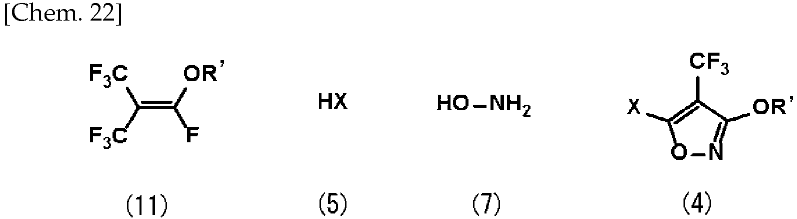
(In the above General Formulae (4), (5), and (11),
R' represents a substituted or unsubstituted hydrocarbon group having 1 to 12 carbon atoms,
X represents a halogen atom, -OA¹, -O-NA¹A², -NA¹(OA²), -NA¹A², -NA³-NA¹A², -NA⁴, or a heterocycle,
A¹, A², and A³ each independently represent a substituted or unsubstituted hydrocarbon group having 1 to 12 carbon atoms,
A⁴ represents an alkylene group represented by =CₙH₂ₙ, and
n is an integer of 1 to 20.)

20. The method for producing a fluorine-containing isoxazole compound according to claim 3, the method comprising:
a step of obtaining a fluorine-containing isoxazole compound represented by General Formula (4-1) below by reacting a fluoroisobutene derivative represented by General Formula (11) below and hydroxylamine represented by General Formula (7) below or a salt thereof. 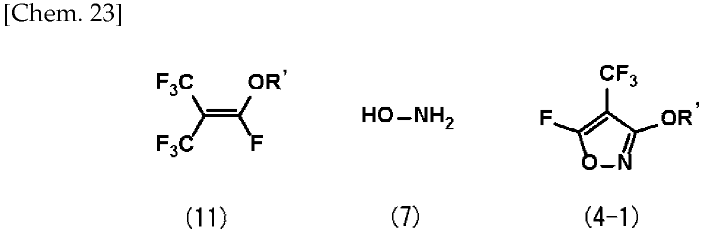
(In the above General Formulae (4-1) and (11),
R' represents a substituted or unsubstituted hydrocarbon group having 1 to 12 carbon atoms.)

21. The method for producing a fluorine-containing isoxazole compound according to claim 3, the method comprising:
a step of obtaining a fluorine-containing isoxazole compound represented by General Formula (4) below by reacting a fluoroisobutane derivative represented by General Formula (12) below, a compound represented by General Formula (5) below or a salt thereof, and hydroxylamine represented by General Formula (7) below or a salt thereof. 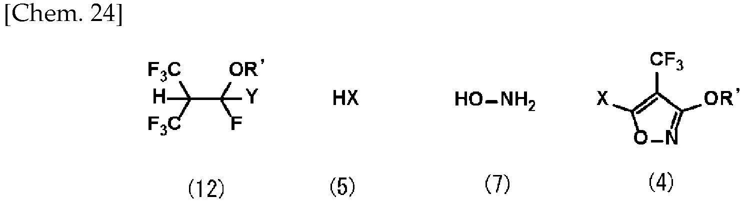
(In the above General Formulae (4), (5), and (12),
R' represents a substituted or unsubstituted hydrocarbon group having 1 to 12 carbon atoms,
X represents a halogen atom, -OA¹, -O-NA¹A², -NA¹A², -NA¹(OA²), -NA³-NA¹A², -NA⁴, or a heterocycle,
Y represents a halogen atom, -OB¹, -SOₘB¹, or -NB¹B²,
m is an integer from 0 to 3,
A¹, A², and A³ each independently represent a substituted or unsubstituted hydrocarbon group having 1 to 12 carbon atoms,
A⁴ represents an alkylene group represented by =CₙH₂ₙ,
n is an integer of 1 to 20, and
B¹ and B² each independently represent a substituted or unsubstituted hydrocarbon group having 1 to 12 carbon atoms.)

22. The method for producing a fluorine-containing isoxazole compound according to claim 3, the method comprising:
a step of obtaining a fluorine-containing isoxazole compound represented by General Formula (4-1) below by reacting a fluoroisobutane derivative represented by General Formula (12) below and hydroxylamine represented by General Formula (7) below. 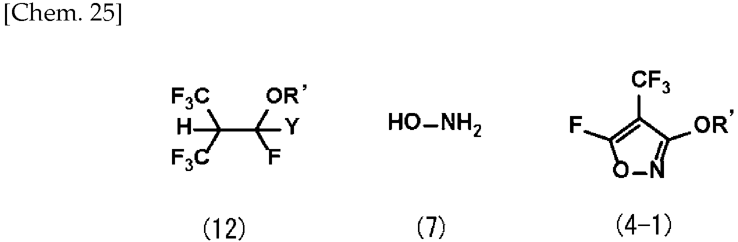
(In the above General Formulae (4-1) and (12),
R' represents a substituted or unsubstituted hydrocarbon group having 1 to 12 carbon atoms,
Y represents a halogen atom, -OB¹, -SOₘB¹, or -NB¹B²,
m is an integer from 0 to 3, and
B¹ and B² each independently represent a substituted or unsubstituted hydrocarbon group having 1 to 12 carbon atoms.)
